# EUROPEAN PATENT APPLICATION

(11) **EP 3 290 047 A1**
(43) Date of publication of application: **07.03.2018**
(21) Application number: 17191200.9
(22) Date of filing: 04.04.2014
(51) Int. Cl.: A61K 38/08, A61K 38/16, C12N 9/50, C12N 9/64

(54) **MATRIX METALLOPROTEINASES AND USES THEREOF**

(30) Priority: 05.04.2013 US 201361808861 P
(62) Divisional of application: 14779257.6
(71) Applicant: Zylberberg, Claudia, Delray Beach, FL 33446 (US); Fields, Gregg B., Palm Beach Gardens, FL 33418 (US)
(72) Inventor: Zylberberg, Claudia, Delray Beach, FL 33446 (US); Fields, Gregg B., Palm Beach Gardens, FL 33418 (US)
(74) Representative: Nuss, Laurent

(57) **Abstract**

Compositions of matrix·metalloproteinases are described. The uses include isolation of cells, in particular stem cells, from tissues, dissociation of tissues, proteins and treatment of a variety of conditions.

## Description

### CROSS REFERENCE TO RELATED APPLICATIONS

This application claims the benefit of U.S. Provisional Application Ser. No 61/808,861 entitled "MATRIX METALLOPROTEINASES AND USES THEREOF", filed April 5, 2013 and is incorporated herein by reference in its entirety.

### FIELD OF THE INVENTION

Embodiments are directed to compositions of matrix metalloproteinases (MMPs), methods of use and therapeutic applications.

### BACKGROUND

Mesenchymal stem cells (MSCs) are known to have the capacity for self-renewal and differentiation into mesenchyme-lineage cell types, including osteoblasts, adipocytes, chondrocytes, tenocytes, and myoblasts, and contribute to the regeneration of a variety of mesenchymal tissues. MSCs can be obtained following a bone marrow aspiration procedure and subsequently cultured *in vitro* without losing their stem cell potential, making them an attractive target for cell therapy. However, traditional bone marrow procurement procedures are distressful for a patient, as they can include pain and morbidity, and usually yield low numbers of MSC upon processing. Adipose tissue, like bone marrow, is derived from the mesenchyme and contains a supportive stroma that is easily isolated. Adipose tissue represents a rich source of mesenchymal stem cells (Zuk PA, et al. Mol. Biol. Cell 2002, 13:4279-4295; Bunnell BA, et al. Methods 2008, 45:115-120), and provides an abundant and accessible source of adult stem cells with minimal patient discomfort. Adipose tissue-derived stem cells (ADSCs) can be isolated from human lipoaspirates, and can be differentiated toward lineages like bone marrow stem cells (BMSCs). Several studies have shown that human ADSCs have similar characteristics to BMSCs *in vitro* and *in vivo.* Thus, adipose tissue may be an ideal source of large amounts of autologous stem cells attainable by a less invasive method than BMSCs.

Isolation of ADSCs has primarily been achieved with Liberase HI (Roche Diagnostics), which is composed of *Clostridium histolyticum* collagenases I and II and thermolysin. All Liberase preparations contain endotoxin. Prior studies have investigated the relative amount of endotoxin in different collagenase preparations and the impact on isolated cell health (Linetsky E. et al., Transplantation Proc. 1998; 30:345-346; Jahr H. et al., J. Mol. Med. (Berl.) 1999;77:118-120; Salamone M, et al., Transplantation Proc. 2010, 42:2043-2048) and found that the presence of endotoxin is harmful for ADSC viability. A solution to the endotoxin contamination problem is the production of recombinant enzymes for use in ADSC isolation. Cell isolation was not as efficient using a single collagenase compared with Liberase HI (Wolters G.H.J. et al., Diabetes 1995; 44:227-234). A significant problem is that collagenase I is the most unstable component of Liberase HI, as the Ia form is rapidly autocatalytically degraded to the Ib form. Degraded collagenases have an adverse effect on islet viability.

### SUMMARY

Embodiments of the invention are directed to compositions comprising one or more matrix metalloproteinases (MMPs), inactive forms of MMPs (e.g. proMMPs), fragments, mutants, variants or combinations thereof. A pharmaceutical composition comprises one or more of the above in a pharmaceutical carrier.

In embodiments, a composition comprises at least one of: a matrix metalloproteinase (MMP), an inactive MMP or a proenzyme (proMMP) thereof, wherein the matrix metalloproteinase (MMPs), inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof.

In preferred embodiments, a composition comprises at least two or more matrix metalloproteinases (MMPs), inactive MMPs or proenzyme (proMMPs) thereof, wherein the matrix metalloproteinase (MMPs), inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof.

In preferred embodiments, a composition comprises three or more matrix metalloproteinases (MMPs), inactive MMPs or proenzyme (proMMPs) thereof, wherein the matrix metalloproteinase (MMPs), inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof.

In preferred embodiments, the MMP, the inactive MMP or a proenzyme (proMMP) comprise: proteins, peptides, polypeptides, nucleic acid sequences, cDNA, ribonucleic acid sequences, chimeric molecules, peptidomimetics, peptide nucleic acids (PNA), or combinations thereof.

In embodiments, a composition comprises a peptide or protein of: a matrix metalloproteinase (MMP), an inactive MMP or a proenzyme (proMMP) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof. In preferred embodiments, the composition further comprises a pharmaceutically acceptable agent, a pharmaceutically acceptable salt or prodrug thereof.

In some embodiments, the composition comprises a peptide or protein of two or more a matrix metalloproteinases (MMPs), inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, variants, mutants, a pharmaceutically acceptable agent, a pharmaceutically acceptable salt or prodrug thereof, or any combinations thereof.

In some embodiments, the composition comprises a peptide or protein of three or more matrix metalloproteinases (MMPs), inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, variants, mutants, a pharmaceutically acceptable agent, a pharmaceutically acceptable salt or prodrug thereof, or any combinations thereof. In some embodiments, the composition comprises a peptide or protein of four or more matrix metalloproteinases (MMPs), inactive MMPs or proenzymes (proMMPs) thereof. In some embodiments, the composition comprises an effective amount of a peptide or protein of five or more matrix metalloproteinases (MMPs), inactive MMPs or proenzymes (proMMPs) thereof.

In some embodiments, a composition comprises a nucleic acid sequence of a matrix metalloproteinase (MMP), an inactive MMP or a proenzyme (proMMP) thereof, wherein the MMP, inactive MMP or proMMP thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, cDNA sequences, mutants, variants, pharmaceutical compositions thereof, a pharmaceutically acceptable salt or prodrug thereof, or any combinations thereof.

In some embodiments, a composition comprises two or more nucleic acids sequence of a matrix metalloproteinase (MMP), an inactive MMPs or a proenzyme (proMMPs) thereof, wherein the matrix metalloproteinases (MMPs), inactive MMPs or a proenzyme (proMMPs) thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, cDNA sequences, mutants, variants, pharmaceutical compositions thereof, a pharmaceutically acceptable salt or prodrug thereof, or any combinations thereof.

In some embodiments, the composition comprises two or more, three or more, four or more nucleic acid sequences of matrix metalloproteinase (MMPs), inactive MMPs or proenzymes (proMMPs) thereof.

In some embodiments, the composition optionally comprises at least one MMP activating agent, at least one MMP inhibitor, or combinations thereof. The at least one MMP activating agent, or the at least one MMP inhibitor, or combinations thereof, are optionally encapsulated for controlled or sustained release over periods of time, physiological conditions, temperatures and the like. In some embodiments, the at least one MMP, at least one MMP activating agent, or at least one MMP inhibitor, or combinations thereof, comprise a controlled release formulation.

In other embodiments, a composition comprising an effective amount of a matrix metalloproteinase (MMP), inactive MMPs or proenzymes (proMMPs) thereof, wherein the MMPs, inactive MMPs or proMMPs thereof, comprise: MMP-1, MMP-2, MMP-3, MMP-8, MMP-9, MMP-11, MMP-12, MMP-13, MMP-19, MMP-25, active fragments, variants, mutants, a pharmaceutically acceptable agent, a pharmaceutically acceptable salt or prodrug thereof, or any combinations thereof. Preferably, the effective amount of any one MMP or proenzyme thereof, catabolizes or dissociates adipose tissue. In another embodiment, an effective amount of any two or more MMPs or proMMPs thereof, catabolizes or dissociate adipose tissue.

In another embodiment, one or more active fragments of one or more MMPs or proMMPs comprising the active fragment, wherein the active fragment catabolizes adipose tissue. In some embodiments, the MMPs are active or inactive or combinations thereof. In another preferred embodiment, the composition, further comprising one or more agents which activate an inactive MMP. In some embodiments, the composition further comprises a pharmaceutically acceptable excipient, a pharmaceutically acceptable salt or prodrug thereof. In embodiments, the matrix metalloproteinase (MMP), inactive MMPs or proenzymes (proMMPs) thereof, comprise: nucleic acid sequences, proteins, polypeptides, peptides, or mutants and variants thereof.

In another preferred embodiment, a method of isolating stem cells from a biological sample, comprising: contacting the biological sample with a composition comprises an effective amount of at least one matrix metalloproteinase (MMP) or inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, or any combinations thereof, wherein the composition catabolizes or dissociates the biological sample, thereby isolating stem cells from the sample. In some embodiments, the composition comprises an effective amount of matrix metalloproteinase (MMP) or inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, or any combinations thereof, wherein the composition catabolizes or dissociates the biological sample, thereby isolating stem cells from the sample. Preferably, the MMPs, inactive MMPs or proMMPs thereof, optionally comprise one or more active fragments of one or more MMPs, inactive MMPs or proMMPs comprising the active fragment. In another preferred embodiment, the method further comprises administering one or more agents which activate the inactive MMPs or fragments thereof. In preferred embodiments, the biological sample comprises: an epithelium, connective tissue, adipose tissue, endothelium, basement membranes, basal lamina, cardiac tissues, endocardium, apical membrane, basolateral membrane, extracellular matrix, dense connective tissue, fibrous connective tissue, olfactory epithelium, loose connective tissue, mucins, mesothelium, stroma, reticular connective tissue, bone marrow, blood, blood vessels, lymphatic tissue, lung, cardiovascular tissue, brain tissue, cerebrospinal tissues and fluids, cerebrovascular tissues and fluids, nervous tissue, brain, bone tissue, skin, muscle, pancreatic tissues, ovarian follicles, cord blood tissue, placenta, intestine lining, brain tissue, spinal tissue, cardiovascular tissue, connective tissue, cerebrospinal fluids or tissue, bone marrow, dermis, blood, periosteum, fibrotic tissue, scar tissue, or any organ tissue.

In another preferred embodiment, a method of treating a subject having a condition associated with excess adipose tissue deposits comprises administering to the subject, a composition comprising an effective amount of at least one matrix metalloproteinase (MMP) inactive MMPs or proenzymes (proMMPs) thereof, wherein the MMPs, inactive MMPs , or proMMPs thereof, comprise: MMP-1, MMP-2, MMP-3, MMP-8, MMP-9, MMP-11, MMP-12, MMP-13, MMP-19, MMP-25, active fragments, mutants, variants, pharmaceutical compositions thereof, a pharmaceutically acceptable salt or prodrug thereof, or any combinations thereof, wherein the composition dissociates or catabolizes the adipose tissue. In some embodiments, a method of treating a subject having a condition associated with excess adipose tissue deposits comprises administering to the subject, a composition comprising an effective amount of two or more matrix metalloproteinase s(MMPs) inactive MMPs or proenzymes (proMMPs) thereof, wherein the MMPs, inactive MMPs, or proMMPs thereof, comprise: MMP-1, MMP-2, MMP-3, MMP-8, MMP-9, MMP-11, MMP-12, MMP-13, MMP-19, MMP-25, active fragments, mutants, variants, pharmaceutical compositions thereof, a pharmaceutically acceptable salt or prodrug thereof, or any combinations thereof, wherein the composition dissociates or catabolizes the adipose tissue. Examples of conditions associated with adipose tissue comprise: cellulite, fat deposits, obesity, metabolic diseases, diabetes, or combinations thereof.

In another preferred embodiments, a method of reducing a regional fat deposit in a subject in need thereof, comprising administering to the subject, a pharmaceutical composition comprising an effective amount of at least one matrix metalloproteinase (MMP), inactive MMPs or proenzymes (proMMPs) thereof, wherein the MMPs, inactive MMPs or proMMPs thereof, comprise: MMP-1, MMP-2, MMP-3, MMP-8, MMP-9, MMP-11, MMP-12, MMP-13 MMP-19, MMP-25, active fragments, mutants, variants, pharmaceutical compositions thereof, a pharmaceutically acceptable salt or prodrug thereof, or any combinations thereof, wherein the regional fat deposit is reduced. In some embodiments the pharmaceutical composition comprises an effective amount of two or more matrix metalloproteinase s (MMPs), inactive MMPs or proenzymes (proMMPs) thereof. In some embodiments, the pharmaceutical composition is administered by a parenteral, topical, intramuscular, subcutaneous, or transdermal route of administration. In some embodiments, the pharmaceutical composition is administered at or near the regional fat deposit.

In another preferred embodiment, a method of isolating stem cells from tissues, comprises contacting a tissue with a composition comprising an effective amount of at least one matrix metalloproteinase (MMP) or inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof, wherein the composition dissociates the tissue, thereby isolating stem cells from the tissue. Preferably, the composition comprises an effective amount of at least two or more, at least three or more, at least four or more, matrix metalloproteinase (MMP) or inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof. In preferred embodiments, the MMPs, inactive MMPs or proMMPs thereof, optionally comprise one or more active fragments of one or more MMPs, inactive MMPs or proMMPs comprising the active fragment. In some embodiments, the MMPs or fragments thereof, are active or inactive or combinations thereof. In some preferred embodiments, the method further comprises administering one or more agents which activate the inactive MMPs or fragments thereof.

In yet another embodiment, a method of treating a patient suffering from a fibrotic disease comprises administering to the patient a composition comprising a therapeutically effective amount of a matrix metalloproteinase (MMP), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions, a pharmaceutically acceptable salt or prodrug thereof, or any combinations thereof.

In another preferred embodiment, an expression vector encoding for at least one matrix metalloproteinase (MMP), inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof.

In another preferred embodiment, an expression vector encoding for two or more matrix metalloproteinases (MMPs), inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof.

In another preferred embodiment, a pharmaceutical composition comprises an expression vector encoding for at least one a matrix metalloproteinase (MMP), inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof.

In another preferred embodiment, a pharmaceutical composition comprises an expression vector encoding for two or more matrix metalloproteinases (MMPs), inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof.

In another preferred embodiment, a time release formulation comprises at least one matrix metalloproteinase (MMP), inactive MMPs or proenzymes (proMMPs) thereof, wherein the MMPs, inactive MMPs or proMMPs, comprise: a matrix metalloproteinase (MMP), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof or any combinations thereof. In another preferred embodiment, a time release formulation comprises two or more matrix metalloproteinases (MMPs), inactive MMPs or proenzymes (proMMPs) thereof, wherein the MMPs, inactive MMPs or proMMPs, comprise: a matrix metalloproteinase (MMP), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof or any combinations thereof. In some embodiments, the time release formulation optionally comprises at least one MMP activating agent, an MMP inhibitor or combinations thereof.

In another preferred embodiment, a method for dissociating a tissue, comprises contacting the tissue with a composition comprising an effective amount of a matrix metalloproteinase (MMP), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions, a pharmaceutically acceptable salt or prodrug thereof or any combinations thereof. In another preferred embodiment, a method for dissociating a tissue, comprises contacting the tissue with a composition comprising an effective amount of two or more matrix metalloproteinases (MMPs), inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions, a pharmaceutically acceptable salt or prodrug thereof or any combinations thereof.

In preferred embodiments, the tissue comprises: an epithelium, connective tissue, adipose tissue, endothelium, basement membranes, basal lamina, cardiac tissues, endocardium, apical membrane, basolateral membrane, extracellular matrix, dense connective tissue, fibrous connective tissue, olfactory epithelium, loose connective tissue, mucins, mesothelium, stroma, reticular connective tissue, bone marrow, blood, blood vessels, lymphatic tissue, lung, cardiovascular tissue, brain tissue, cerebrospinal tissues and fluids, cerebrovascular tissues and fluids, nervous tissue, brain, bone tissue, skin, muscle, pancreatic tissues, ovarian follicles, cord blood tissue, placenta, intestine lining, brain tissue, spinal tissue, cardiovascular tissue, connective tissue, cerebrospinal fluids or tissue, bone marrow, dermis, blood, periosteum, fibrotic tissue, scar tissue, or any organ tissue.

In another preferred embodiment, a method of dissociating a protein matrix, comprises contacting a protein matrix with a composition comprising an effective amount of a matrix metalloproteinase (MMP), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions, a pharmaceutically acceptable salt or prodrug thereof or any combinations thereof. In another preferred embodiment, a method of dissociating a protein matrix, comprises contacting a protein matrix with a composition comprising an effective amount of two or more matrix metalloproteinases (MMPs), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions, a pharmaceutically acceptable salt or prodrug thereof or any combinations thereof.

In preferred embodiments, the protein matrix comprises: collagen, fibronectin, gelatin, laminin, aggregan, elastin, fibrin, fibrinogen, or combinations thereof.

In other preferred embodiments, a method of treating or healing a scar or a wound comprises contacting scar tissue or wound with a composition comprising at least one: a matrix metalloproteinase (MMP), an inactive MMP or a proenzyme (proMMP) thereof, wherein the matrix metalloproteinase (MMPs), inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof. Preferably, the MMP, the inactive MMP or a proenzyme (proMMP) comprise: proteins, peptides, polypeptides, nucleic acid sequences, cDNA, ribonucleic acid sequences, chimeric molecules, peptidomimetics, peptide nucleic acids (PNA), or combinations thereof. Preferably, the composition further comprises a pharmaceutically acceptable agent, a pharmaceutically acceptable salt or prodrug thereof. In other preferred embodiments, the composition comprises an effective amount of any two or more MMPs or proMMPs, active fragments, variants, mutants, or any combinations thereof, dissociates or catabolizes the scar tissue or wound.

In another preferred embodiment, a method of dissociating fibrotic tissue comprises contacting the fibrotic tissue with a composition comprising at least one: a matrix metalloproteinase (MMP), an inactive MMP or a proenzyme (proMMP) thereof, wherein the matrix metalloproteinase (MMPs), inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof. Preferably, the MMP, the inactive MMP or a proenzyme (proMMP) comprise: proteins, peptides, polypeptides, nucleic acid sequences, cDNA, ribonucleic acid sequences, chimeric molecules, peptidomimetics, peptide nucleic acids (PNA), or combinations thereof. Preferably, the composition further comprises a pharmaceutically acceptable agent, a pharmaceutically acceptable salt or prodrug thereof. In other preferred embodiments, the composition comprises an effective amount of any two or more MMPs or proMMPs, active fragments, variants, mutants, or any combinations thereof, dissociates or catabolizes the fibrotic tissue.

In another preferred embodiment, a kit comprises at least one matrix metalloproteinase (MMP), inactive MMPs or proenzymes (proMMPs) thereof, wherein the MMPs, inactive MMPs or proMMPs, comprise: a matrix metalloproteinase (MMP), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof.

In some embodiments, the kit further comprises at least one MMP activating agent or at least one MMP inhibitor, or combinations thereof.

Other aspects, objectives and advantages of this invention will become apparent from the following description taken in conjunction with the accompanying drawings wherein are set forth, by way of illustration and example, certain embodiments of this invention. The drawings constitute a part of this specification and include exemplary embodiments of the present invention and illustrate various objects and features thereof.

### BRIEF DESCRIPTION OF THE DRAWINGS

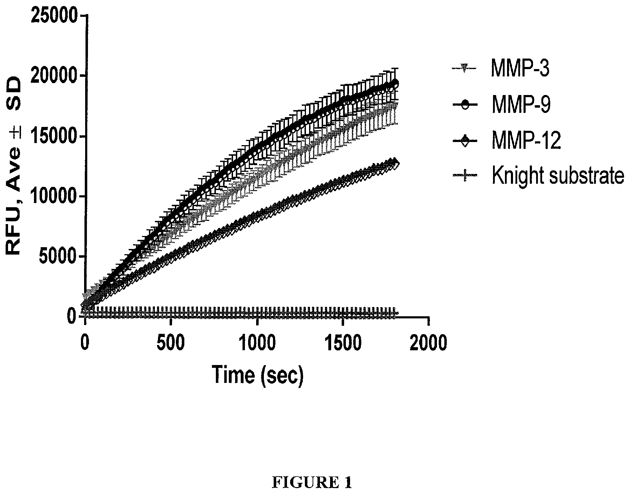

Figure 1 is a graph showing recombinant enzyme activation. MMP-3 was activated with 5 µg/ ml chymotrypsin for 30 minutes at 37°C (light blue), while MMP-12 was self-activated in TSB overnight at 37°C. MMP-9 was purchased in an activated form. Enzyme activity was tested with 5 µM Knight substrate over 30 minutes.

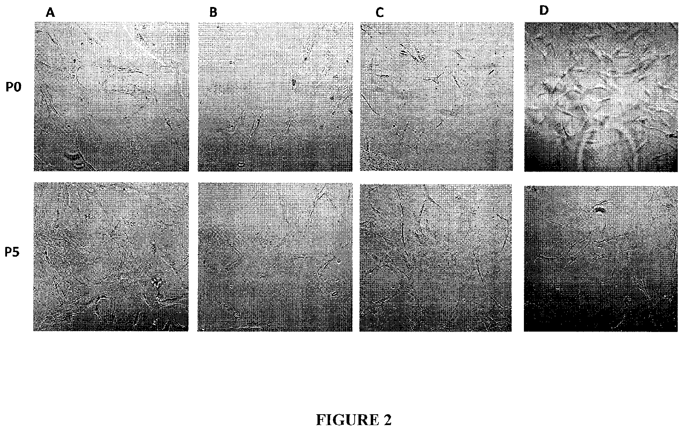

Figure 2 are scans of photographs showing the morphology of isolated MSCs. Morphology of MSCs freshly isolated from the adipose tissue and grown in tissue culture dish for 6-7 days (Passage 0, top row) and at passage 5 (bottom row) did not display any obvious differences between LIBERASE™ (A), MMP-3 (B), MMP-12 (C) and Collagenase I (D) isolated cells.

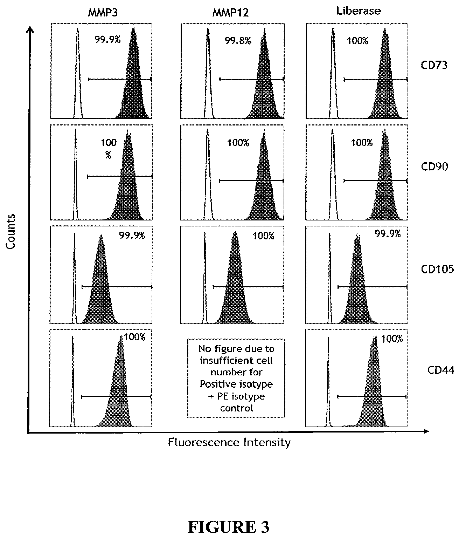

Figure 3 shows the immunophenotyping of MSCs by flow cytometry. Detection of surface markers expressed by LIBERASE™, MMP-3 and MMP-12-isolated MSCs. Clear histograms represent cells stained with isotype control antibodies, and filled histograms represent the staining with specific marker. The percentage of positive cells is shown in each panel.

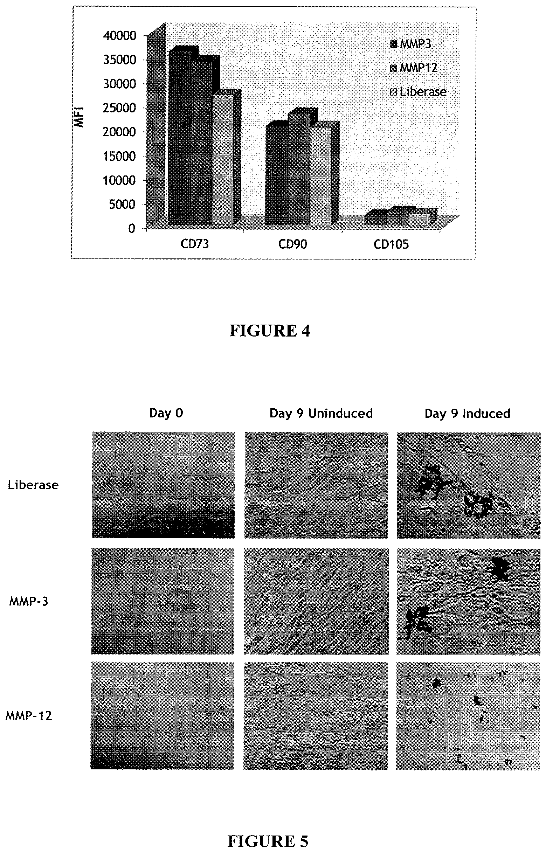

Figure 4 is a graph showing the Median Fluorescence Intensity (MFI) of CD73, CD90 and CD105 on MSCs isolated by LIBERASE™, MMP-3 and MMP-12.
Figure 5 is a scan of a photograph showing the histochemical staining of MSCs induced into adipocytes (Oil Red O). MSCs were induced into adipogenesis for 10 days. Oil droplets appear as black dots in "Day 9 induced" panel.

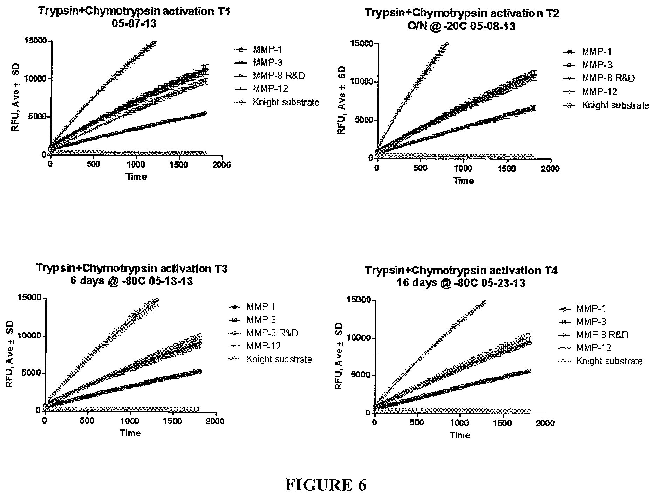

Figure 6 is a series of graphs showing enzyme activation following enzyme storage by freezing. **All enzymes activated for 30 min. Headers indicate the amounts of time enzymes were frozen, while the x-axis indicates the amount of time measuring enzyme activity.

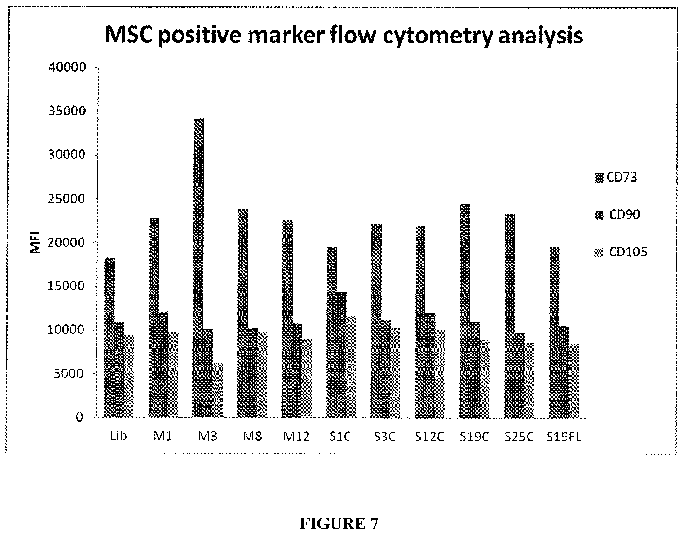

Figure 7 is a graph showing the results of flow cytometry for MSC positive markers. *** Liberase-isolated MSC sample contained 6% non-MSC cells (PE-negative control), while all others contained <1% of non-MSC cells. *** MSCs were isolated by incubating adipose tissue with LIBERASE™ at 13 Wünsch Units/ml and 400 ng/ml of each MMP for 30 minutes in 37°C on a rotating platform. Lib = LIBERASE™, M1 = MMP-1, M3 = MMP-3, M8 = MMP-8, M12 = MMP-12, S1C = MMP-1 catalytic domain, S3C = MMP-3 catalytic domain, S12C = MMP-12 catalytic domain, S19C = MMP-19 catalytic domain, S25C = MMP-25 catalytic domain, and S19FL = MMP-19.

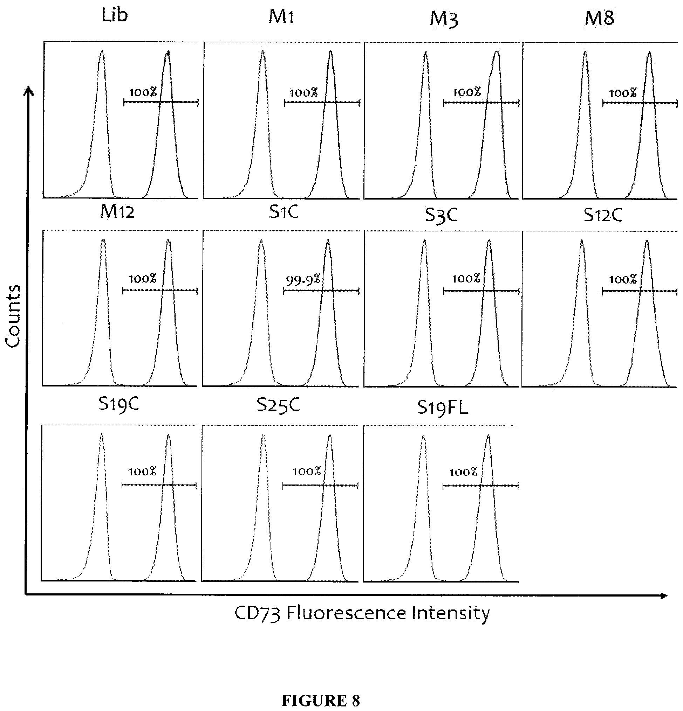

Figure 8 is a graph showing a comparison of results of flow cytometry obtained for isolation of stem cells using MMPs and LIBERASE™ as measured by CD73 intensity. Lib = LIBERASE™, M1 = MMP-1, M3 = MMP-3, M8 = MMP-8, M12 = MMP-12, S1C = MMP-1 catalytic domain, S3C = MMP-3 catalytic domain, S12C = MMP-12 catalytic domain, S19C = MMP-19 catalytic domain, S25C = MMP-25 catalytic domain, and S19FL = MMP-19.

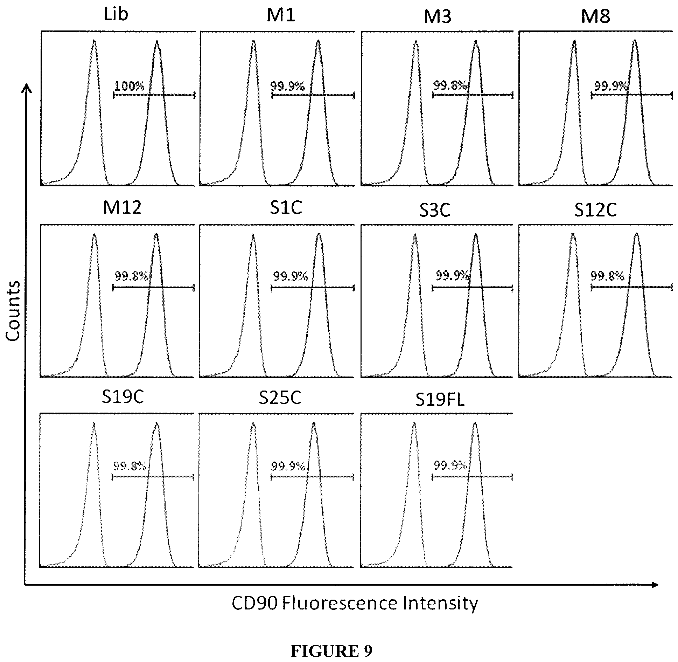

Figure 9 is a graph showing a comparison of results of flow cytometry obtained for isolation of stem cells using MMPs and LIBERASE™ as measured by CD90 intensity. Lib = LIBERASE™, M1 = MMP-1, M3 = MMP-3, M8 = MMP-8, M12 = MMP-12, S1C = MMP-1 catalytic domain, S3C = MMP-3 catalytic domain, S12C = MMP-12 catalytic domain, S19C = MMP-19 catalytic domain, S25C = MMP-25 catalytic domain, and S19FL = MMP-19.

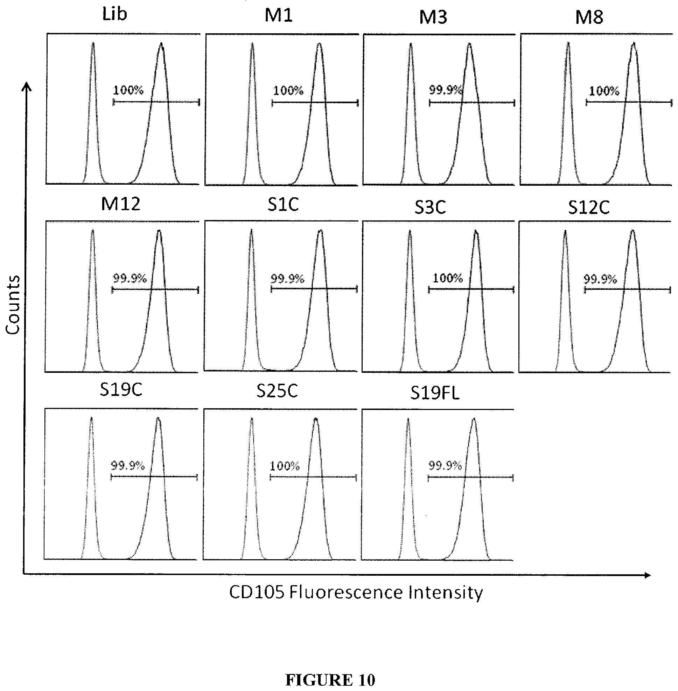

Figure 10 is a graph showing a comparison of results of flow cytometry obtained for isolation of stem cells using MMPs and LIBERASE™ as measured by CD105 intensity. Lib = LIBERASE™, M1 = MMP-1, M3 = MMP-3, M8 = MMP-8, M12 = MMP-12, S1C = MMP-1 catalytic domain, S3C = MMP-3 catalytic domain, S12C = MMP-12 catalytic domain, S19C = MMP-19 catalytic domain, S25C = MMP-25 catalytic domain, and S19FL = MMP-19.

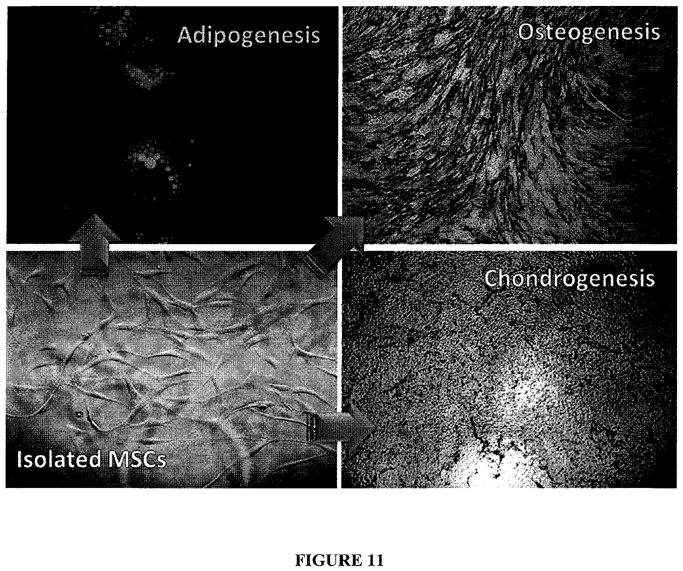

Figure 11 is a scan of micrographs showing that the MSCs isolated using MMP-12 catalytic domain can be induced to differentiate into various lineages (adipocytes, osteoblasts, and chondrocytes).

### DETAILED DESCRIPTION

Isolation of ADSCs has primarily been achieved with LIBERASE™ (Roche Diagnostics), which is composed of *Clostridium histolyticum* collagenases I and II and thermolysin. Crude preparations from *Clostridium histolyticum* contain not only several collagenases but also a sulhydryl protease, clostripain, a trypsin-like enzyme, and an aminopeptidase. During LIBERASE™ enzyme production, collagenase isoenzymes are purified by a process that removes a significant amount of the endotoxin present in the raw material. There is a wide range of endotoxin contamination of traditional collagenase preparations compared with the endotoxin level of LIBERASE™. However, all LIBERASE™ preparations contain endotoxin. Thus, regardless of the source, all purified collagenases and neutral proteases from bacterial bullion are contaminated with endotoxin. Prior studies have investigated the relative amount of endotoxin in different collagenase preparations and the impact on isolated cell health, the presence of endotoxin is harmful for ADSC viability.

The advantages of the present invention are many fold, because without the highest quality of connective tissue degrading enzymes it is virtually impossible to liberate viable ADSCs with good and stable proliferative capabilities. Success in cell transplantation is directly proportional to quality of stem cells isolated, cells cultivated, and allografts prepared. The compositions and methods embodied herein will be of enormous benefit for public health. First, a breakthrough in the entire field of the MSCs isolation and transplantation technology is achieved. Second, collection of MSCs of highest quality with a long time of life expectancy. Third, by abolishing toxicity, a standard protocol for adipose tissue processing and MSCs isolation is provided. Forth, a highly purified new recombinant MMP- cocktail will be provided for tissue dissociation practice (not only for adipose tissue dissolution), replacing current collagenases of microbial origin and becoming a new standard. Fifth, the compositions are useful in methods of treatment and cosmetic procedures. Other benefits will be apparent from the description.

The following description of the preferred embodiments is merely exemplary in nature and is in no way intended to limit the invention, its application or uses. Embodiments of the invention may be practiced without the theoretical aspects presented. Moreover, the theoretical aspects are presented with the understanding that Applicants do not seek to be bound by the theory presented.

It should be understood that numerous specific details, relationships, and methods are set forth to provide a full understanding of the invention. One having ordinary skill in the relevant art, however, will readily recognize that the invention can be practiced without one or more of the specific details or with other methods. The present invention is not limited by the illustrated ordering of acts or events, as some acts may occur in different orders and/or concurrently with other acts or events. Furthermore, not all illustrated acts or events are required to implement a methodology in accordance with the present invention.

All genes, gene names, and gene products disclosed herein are intended to correspond to homologs from any species for which the compositions and methods disclosed herein are applicable. Thus, the terms include, but are not limited to genes and gene products from humans and mice. It is understood that when a gene or gene product from a particular species is disclosed, this disclosure is intended to be exemplary only, and is not to be interpreted as a limitation unless the context in which it appears clearly indicates. Thus, for example, for the genes or gene products disclosed herein, which in some embodiments relate to mammalian nucleic acid and amino acid sequences, are intended to encompass homologous and/or orthologous genes and gene products from other animals including, but not limited to other mammals, fish, amphibians, reptiles, and birds. In preferred embodiments, the genes, nucleic acid sequences, amino acid sequences, peptides, polypeptides and proteins are human.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

### Definitions

The terminology used herein is for the purpose of describing particular embodiments only and is not intended to be limiting of the invention. As used herein, the singular forms "a", "an" and "the" are intended to include the plural forms as well, unless the context clearly indicates otherwise. Furthermore, to the extent that the terms "including", "includes", "having", "has", "with", or variants thereof are used in either the detailed description and/or the claims, such terms are intended to be inclusive in a manner similar to the term "comprising."

As used herein, the terms "comprising," "comprise" or "comprised," and variations thereof, in reference to defined or described elements of an item, composition, apparatus, method, process, system, etc. are meant to be inclusive or open ended, permitting additional elements, thereby indicating that the defined or described item, composition, apparatus, method, process, system, etc. includes those specified elementsor, as appropriate, equivalents thereof--and that other elements can be included and still fall within the scope/definition of the defined item, composition, apparatus, method, process, system, etc.

The term "about" or "approximately" means within an acceptable error range for the particular value as determined by one of ordinary skill in the art, which will depend in part on how the value is measured or determined, *i.e.,* the limitations of the measurement system. For example, "about" can mean within 1 or more than 1 standard deviation, per the practice in the art. Alternatively, "about" can mean a range of up to 20%, preferably up to 10%, more preferably up to 5%, and more preferably still up to 1% of a given value. Alternatively, particularly with respect to biological systems or processes, the term can mean within an order of magnitude, preferably within 5-fold, and more preferably within 2-fold, of a value. Where particular values are described in the application and claims, unless otherwise stated the term "about" meaning within an acceptable error range for the particular value should be assumed.

"Optional" or "optionally" means that the subsequently described event or circumstance can or cannot occur, and that the description includes instances where the event or circumstance occurs and instances where it does not.

As used herein, unless otherwise indicated, the terms "peptide", "polypeptide" or "protein" are used interchangeably herein, and refer to a polymer of amino acids of varying sizes, e.g. fragments of MMPs. These terms do not connote a specific length of a polymer of amino acids. Thus, for example, the terms oligopeptide, protein, and enzyme are included within the definition of polypeptide or peptide, whether produced using recombinant techniques, chemical or enzymatic synthesis, or be naturally occurring. This term also includes polypeptides that have been modified or derivatized, such as by glycosylation, acetylation, phosphorylation, and the like.

As used herein, a "nucleic acid" or "nucleic acid sequence" or "cDNA" refers to a nucleic acid segment or fragment which has been separated from sequences which flank it in a naturally occurring state, e.g., a DNA fragment which has been removed from the sequences which are normally adjacent to the fragment, e.g., the sequences adjacent to the fragment in a genome in which it naturally occurs, and refers to nucleic acid sequences in which one or more introns have been removed. The term also applies to nucleic acids which have been substantially purified from other components which naturally accompany the nucleic acid, e.g., RNA or DNA or proteins, which naturally accompany it in the cell. The term therefore includes, for example, a recombinant DNA which is incorporated into a vector, into an autonomously replicating plasmid or virus, or into the genomic DNA of a prokaryote or eukaryote, or which exists as a separate molecule (e.g., as a cDNA or a genomic or cDNA fragment produced by PCR or restriction enzyme digestion) independent of other sequences. It also includes a recombinant DNA, for instance, DNA which is part of a hybrid gene encoding additional polypeptide sequences.

A "polynucleotide" means a single strand or parallel and anti-parallel strands of a nucleic acid. Thus, a polynucleotide may be either a single-stranded or a double-stranded nucleic acid.

The term "variant," when used in the context of a polynucleotide sequence, may encompass a polynucleotide sequence related to a wild type gene. This definition may also include, for example, "allelic," "splice," "species," or "polymorphic" variants. A splice variant may have significant identity to a reference molecule, but will generally have a greater or lesser number of polynucleotides due to alternate splicing of exons during mRNA processing. The corresponding polypeptide may possess additional functional domains or an absence of domains. Species variants are polynucleotide sequences that vary from one species to another. Of particular utility in the invention are variants of wild type gene products. Variants may result from at least one mutation in the nucleic acid sequence and may result in altered mRNAs or in polypeptides whose structure or function may or may not be altered. Any given natural or recombinant gene may have none, one, or many allelic forms. Common mutational changes that give rise to variants are generally ascribed to natural deletions, additions, or substitutions of nucleotides. Each of these types of changes may occur alone, or in combination with the others, one or more times in a given sequence.

"Encoding" refers to the inherent property of specific sequences of nucleotides in a polynucleotide, such as a gene, a cDNA, or an mRNA, to serve as templates for synthesis of other polymers and macromolecules in biological processes having either a defined sequence of nucleotides (i.e., rRNA, tRNA and mRNA) or a defined sequence of amino acids and the biological properties resulting therefrom. Thus, a gene encodes a protein if transcription and translation of mRNA corresponding to that gene produces the protein in a cell or other biological system. Both the coding strand, the nucleotide sequence of which is identical to the mRNA sequence and is usually provided in sequence listings, and the non-coding strand, used as the template for transcription of a gene or cDNA, can be referred to as "encoding" the protein or other product of that gene or cDNA. By "encoding" or "encoded", "encodes", with respect to a specified nucleic acid, is meant comprising the information for translation into the specified protein. A nucleic acid encoding a protein may comprise non-translated sequences (e.g., introns) within translated regions of the nucleic acid, or may lack such intervening non-translated sequences (e.g., as in cDNA). The information by which a protein is encoded is specified by the use of codons. Typically, the amino acid sequence is encoded by the nucleic acid using the "universal" genetic code.

Unless otherwise specified, a "nucleotide sequence encoding an amino acid sequence" includes all nucleotide sequences that are degenerate versions of each other and that encode the same amino acid sequence. Nucleotide sequences that encode proteins and RNA may include introns.

As used herein, "proMMP" is used to mean a protein obtained as a result of expression of the pro form of a matrix metalloproteinase (also known as a matrix metalloprotease). Within the meaning of this term, it will be understood that a proMMP encompasses all proteins encoded by a proMMP gene or cDNA, mutants thereof, including deletions, substitutions, and truncations, as well as modified forms thereof. As used herein, the term "proMMP" also includes partially processed forms of a proMMP that have not yet been completely processed to the active form.

As used herein, "matrix metalloproteinase" (MMP), e.g. MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, refers to any protein, peptide, or polypeptide having any MMP activity. The term "MMP" also refers to nucleic acid sequences encoding any MMP- protein, peptide, or polypeptide having MMP activity. The term "MMP" is also meant to include other MMP encoding sequences, such as other MMP isoforms, mutant MMP genes, splice variants of MMP genes, and MMP gene polymorphisms. Thus, for example MMP-12 is also meant to include other MMP-12 encoding sequences, such as other MMP-12 isoforms, mutant MMP-12 genes, splice variants of MMP-12 genes, and MMP-12 gene polymorphisms.

As used herein, the term "activation" refers to the processing that occurs to change from an inactive pro form of a matrix metalloproteinase (proMMP) to an active form of a matrix metalloproteinase (MMP). An "MMP activating agent" is a molecule which activates or converts to an active form, the MMP which it is specific for. Conversely, an "MMP inhibitor" is a molecule which suppresses or inhibits, or renders the MMP which it specifically acts upon, inactive.

As used herein, the term "activity" or "active form" refers to an activity exerted by a matrix metalloproteinase (MMP) as determined *in vivo* or *in vitro,* according to standard techniques. The term "active fragment" contains the active site or segments of the molecule that confer activity. Examples of such activity include, but are not limited to, direct activity such as catalytic activity of the extracellular matrix components (e.g. collagens, laminin, fibronectin, elastin etc.) or the ability to bind to a ligand or an analog thereof, changes in transcriptional activity or changes in the levels of genes or gene products that are regulated directly or indirectly by MMP- activity, changes in enzymatic activity for another protein whose expression may be affected directly or indirectly by MMP- activity, or functional changes of cell physiology that result from changes in MMP- activity.

As used herein, the term "active site" refers to regions on an active MMP or a structural motif of an active MMP that are directly involved in the catalytic activity of animal tissues, e.g. adipose. In preferred embodiments, the animal is human. Inclusion of polypeptides with an amino acid sequence having at least 90% identity, and more preferably 95% identity, to the polypeptide sequence of a characterized matrix metalloproteinase is intended to cover closely related forms of the MMP, such as those that include minor mutations or other changes, but retain enzymatic activity. The similarity is referred to as structural similarity, and is generally determined by aligning the residues of a candidate polypeptide with the sequence of interest. For example, with MMP-12, a candidate MMP-12 enzyme amino acid sequence is aligned with a known sequence of MMP-12 to optimize the number of identical amino acids along the lengths of their sequences. Gaps in either or both sequences are permitted in making the alignment in order to optimize the number of identical amino acid sequences, but the amino acids in each sequence should remain in their proper order. Preferably, two amino acid sequences are compared using the Blastp program of the BLAST 2 search algorithm, as described by Tatusova, et al. (FEMS Microbiol. Lett, 174:247-250 (1999)).

As used herein, the terms "nucleic acid sequence", "polynucleotide," and "gene" are used interchangeably throughout the specification and include complementary DNA (cDNA), linear or circular oligomers or polymers of natural and/or modified monomers or linkages, including deoxyribonucleosides, ribonucleosides, substituted and alpha-anomeric forms thereof, peptide nucleic acids (PNA), locked nucleic acids (LNA), phosphorothioate, methylphosphonate, and the like.

The nucleic acid sequences may be "chimeric," that is, composed of different regions. In the context of this invention "chimeric" compounds are oligonucleotides, which contain two or more chemical regions, for example, DNA region(s), RNA region(s), PNA region(s) etc. Each chemical region is made up of at least one monomer unit, i.e., a nucleotide. These sequences typically comprise at least one region wherein the sequence is modified in order to exhibit one or more desired properties.

As used herein, the term "monomers" typically indicates monomers linked by phosphodiester bonds or analogs thereof to form oligonucleotides ranging in size from a few monomeric units, e.g., from about 3-4, to about several hundreds of monomeric units. Analogs of phosphodiester linkages include: phosphorothioate, phosphorodithioate, methylphosphornates, phosphoroselenoate, phosphoramidate, and the like, as more fully described below.

In the present context, the terms "nucleobase" covers naturally occurring nucleobases as well as non-naturally occurring nucleobases. It should be clear to the person skilled in the art that various nucleobases which previously have been considered "non-naturally occurring" have subsequently been found in nature. Thus, "nucleobase" includes not only the known purine and pyrimidine heterocycles, but also heterocyclic analogues and tautomers thereof. Illustrative examples of nucleobases are adenine, guanine, thymine, cytosine, uracil, purine, xanthine, diaminopurine, 8-oxo-N6-methyladenine, 7-deazaxanthine, 7-deazaguanine, N4,N4-ethanocytosin, N6,N6-ethano-2,6-diaminopurine, 5-methylcytosine, 5-(C3-C6)-alkynylcytosine, 5-fluorouracil, 5-bromouracil, pseudoisocytosine, 2-hydroxy-5-methyl-4-triazolopyridin, isocytosine, isoguanin, inosine and the "non-naturally occurring" nucleobases described in Benner et al., U.S. Pat No. 5,432,272. The term "nucleobase" is intended to cover every and all of these examples as well as analogues and tautomers thereof. Especially interesting nucleobases are adenine, guanine, thymine, cytosine, and uracil, which are considered as the naturally occurring nucleobases in relation to therapeutic and diagnostic application in humans.

As used herein, "nucleoside" includes the natural nucleosides, including 2'-deoxy and 2'-hydroxyl forms, e.g., as described in Kornberg and Baker, DNA Replication, 2nd Ed. (Freeman, San Francisco, 1992).

"Analogs" in reference to nucleosides includes synthetic nucleosides having modified base moieties and/or modified sugar moieties, e.g., described generally by Scheit, Nucleotide Analogs, John Wiley, New York, 1980; Freier & Altmann, Nucl. Acid. Res., 1997, 25(22), 4429-4443, Toulmé, J.J., Nature Biotechnology 19:17-18 (2001); Manoharan M., Biochemica et Biophysica Acta 1489:117-139(1999); Freier S. M., Nucleic Acid Research, 25:4429-4443 (1997), Uhlman, E., Drug Discovery & Development, 3: 203-213 (2000), Herdewin P., Antisense & Nucleic Acid Drug Dev., 10:297-310 (2000), ); 2'-O, 3'-C-linked [3.2.0] bicycloarabinonucleosides (see e.g. N.K Christiensen., et al, J. Am. Chem. Soc., 120: 5458-5463 (1998). Such analogs include synthetic nucleosides designed to enhance binding properties, e.g., duplex or triplex stability, specificity, or the like.

The term "variant," when used in the context of a polynucleotide sequence, may encompass a polynucleotide sequence related to a wild type gene. This definition may also include, for example, "allelic," "splice," "species," or "polymorphic" variants. A splice variant may have significant identity to a reference molecule, but will generally have a greater or lesser number of polynucleotides due to alternate splicing of exons during mRNA processing. The corresponding polypeptide may possess additional functional domains or an absence of domains. Species variants are polynucleotide sequences that vary from one species to another. Of particular utility in the invention are variants of wild type target gene products. Variants may result from at least one mutation in the nucleic acid sequence and may result in altered mRNAs or in polypeptides whose structure or function may or may not be altered. Any given natural or recombinant gene may have none, one, or many allelic forms. Common mutational changes that give rise to variants are generally ascribed to natural deletions, additions, or substitutions of nucleotides. Each of these types of changes may occur alone, or in combination with the others, one or more times in a given sequence.

The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs,) or single base mutations in which the polynucleotide sequence varies by one base. The presence of SNPs may be indicative of, for example, a certain population with a propensity for a disease state, that is susceptibility versus resistance.

As used herein, "variant" of polypeptides refers to an amino acid sequence that is altered by one or more amino acid residues. The variant may have "conservative" changes, wherein a substituted amino acid has similar structural or chemical properties (e.g., replacement of leucine with isoleucine). More rarely, a variant may have "nonconservative" changes (e.g., replacement of glycine with tryptophan). Analogous minor variations may also include amino acid deletions or insertions, or both. Guidance in determining which amino acid residues may be substituted, inserted, or deleted without abolishing biological activity may be found using computer programs well known in the art, for example, LASERGENE software (DNASTAR).

As used herein, the term "tissue" refers to an aggregate of cells together with their extracellular substances that form one of the structural or other materials of a patient. As used herein, the term "tissue" is intended to include any tissue of the body including but not limited to capillaries, blood vessels, muscle and organ tissue, wound tissue, tumor tissue, bone tissue, or cartilage tissue. Also, the term "tissue" as used herein may also refer to an individual cell.

In the present context, "stromal vascular fraction (SVF)" refers to cells isolated from adipose tissue and means a remaining group of cells after removing most of mature adipocytes by treating adipose tissue with the compositions embodied herein.

"Adipose-derived stromal stem cells" mean mesenchymal stem cells obtained from the SVF and may also be designated as "adipose-derived stem cells (ASC)," "adipose-derived stem cells (ADSC)," "adipose stem cells," or the like. Adipose-derived stromal stem cells in the present invention may be isolated from human subcutaneous fat tissue by liposuction or surgical excision, without being particularly limited thereto.

"Bone marrow derived progenitor cell" (BMDC) or "bone marrow derived stem cell" refers to a primitive stem cell with the machinery for self-renewal constitutively active. Included in this definition are stem cells that are totipotent, pluripotent and precursors. A "precursor cell" can be any cell in a cell differentiation pathway that is capable of differentiating into a more mature cell. As such, the term "precursor cell population" refers to a group of cells capable of developing into a more mature cell. A precursor cell population can comprise cells that are totipotent, cells that are pluripotent and cells that are stem cell lineage restricted (i.e. cells capable of developing into less than all hematopoietic lineages, or into, for example, only cells of erythroid lineage). As used herein, the term "totipotent cell" refers to a cell capable of developing into all lineages of cells. Similarly, the term "totipotent population of cells" refers to a composition of cells capable of developing into all lineages of cells. Also as used herein, the term "pluripotent cell" refers to a cell capable of developing into a variety (albeit not all) lineages and are at least able to develop into all hematopoietic lineages (e.g., lymphoid, erythroid, and thrombocytic lineages). Bone marrow derived stem cells contain two well-characterized types of stem cells. Mesenchymal stem cells (MSC) normally form chondrocytes and osteoblasts. Hematopoietic stem cells (HSC) are of mesodermal origin that normally give rise to cells of the blood and immune system (e.g., erythroid, granulocyte/macrophage, magakaryocite and lymphoid lineages). In addition, hematopoietic stem cells also have been shown to have the potential to differentiate into the cells of the liver (including hepatocytes, bile duct cells), lung, kidney (e.g., renal tubular epithelial cells and renal parenchyma), gastrointestinal tract, skeletal muscle fibers, astrocytes of the CNS, Purkinje neurons, cardiac muscle (e.g., cardiomyocytes), endothelium and skin. As used herein, the term "stem cell" is a general term and is meant to be inclusive of all types of stem cells.

"Isolating" a cell or a stem cell refers to the process of removing a cell or stem cell from a tissue sample and separating away other cells which are not the desired cell or stem cells of the tissue. An isolated stem cell will be generally free from contamination by other cell types and will generally have the capability of propagation and differentiation to produce mature cells of the tissue from which it was isolated. However, when dealing with a collection of stem cells, e.g., a culture of stem cells, it is understood that it is practically impossible to obtain a collection of stem cells which is 100% pure. Therefore, an isolated stem cell can exist in the presence of a small fraction of other cell types which do not interfere with the utilization of the stem cell for analysis or production of other, differentiated cell types. Isolated stem cells will generally be at least 30%, 40%, 50%, 60%, 70%, 80%, 85%, 90%, 95%, 98%, or 99% pure. Preferably, isolated stem cells according to the invention will be at least 98% or at least 99% pure. The stem cells can be isolated from any tissue. In some embodiments, the tissue is adipose tissue.

"Biological samples" include solid (e.g. tissues, organs) and body fluid samples. The biological samples used in the present invention can include cells, protein or membrane extracts of cells, blood or biological fluids such as ascites fluid or brain fluid (e.g., cerebrospinal fluid). Examples of solid biological samples include, but are not limited to, samples taken from tissues of the central nervous system, bone, breast, kidney, cervix, endometrium, head/neck, gallbladder, parotid gland, prostate, pituitary gland, muscle, esophagus, stomach, small intestine, colon, liver, spleen, pancreas, thyroid, heart, lung, bladder, adipose, lymph node, uterus, ovary, adrenal gland, testes, tonsils and thymus. Examples of "body fluid samples" include, but are not limited to blood, serum, semen, prostate fluid, seminal fluid, urine, saliva, sputum, mucus, bone marrow, lymph, and tears.

The term "transplant" includes any cell, organ, organ system or tissue which can elicit an immune response in a recipient subject mammal. In general, therefore, a transplant includes an allograft or a xenograft cell, organ, organ system or tissue. An allograft refers to a graft (cell, organ, organ system or tissue) obtained from a member of the same species as the recipient. A xenograft refers to a graft (cell, organ, organ system or tissue) obtained from a member of a different species as the recipient.

"Treatment" is an intervention performed with the intention of preventing the development or altering the pathology or symptoms of a disorder. Accordingly, "treatment" refers to both therapeutic treatment and prophylactic or preventative measures. "Treatment" may also be specified as palliative care. Those in need of treatment include those already with the disorder as well as those in which the disorder is to be prevented. Accordingly, "treating" or "treatment" of a state, disorder or condition includes: (1) preventing or delaying the appearance of clinical symptoms of the state, disorder or condition developing in a human or other mammal that may be afflicted with or predisposed to the state, disorder or condition but does not yet experience or display clinical or subclinical symptoms of the state, disorder or condition; (2) inhibiting the state, disorder or condition, *i.e.,* arresting, reducing or delaying the development of the disease or a relapse thereof (in case of maintenance treatment) or at least one clinical or subclinical symptom thereof; or (3) relieving the disease, *i.e.,* causing regression of the state, disorder or condition or at least one of its clinical or subclinical symptoms. The benefit to an individual to be treated is either statistically significant or at least perceptible to the patient or to the physician.

The terms "patient" or "individual" or "subject" are used interchangeably herein, and refers to a mammalian subject to be treated, with human patients being preferred. In some cases, the methods of the invention find use in experimental animals, in veterinary application, and in the development of animal models for disease, including, but not limited to, rodents including mice, rats, and hamsters, and primates.

As defined herein, a "therapeutically effective" amount of a compound or agent (i.e., an effective dosage) means an amount sufficient to produce a therapeutically (e.g., clinically) desirable result. The compositions can be administered from one or more times per day to one or more times per week; including once every other day. The skilled artisan will appreciate that certain factors can influence the dosage and timing required to effectively treat a subject, including but not limited to the severity of the disease or disorder, previous treatments, the general health and/or age of the subject, and other diseases present. Moreover, treatment of a subject with a therapeutically effective amount of the compounds of the invention can include a single treatment or a series of treatments.

As defined herein, an "effective" amount of a compound or agent (i.e., an effective dosage) means an amount sufficient to produce a (e.g., clinically) desirable result. In some embodiments, the desired result is the degradation of adipose tissue or fat deposits. In other embodiments, the desired result is isolation of stem cells from a variety of tissues, bone marrow, organs. In other embodiments, the desired result is islet isolation from pancreas. In other embodiments, the desired result is isolation of viable follicles from human ovarian tissue. In other embodiments, the desired result is tissue dissociation to isolate a variety of cell types, including cardiac myocytes, fibroblasts, and dendritic cells.

The term "pharmaceutically acceptable prodrugs" as used herein refers to those prodrugs of the MMP compounds formed by the process of the present invention which are, within the scope of sound medical judgment, suitable for use in contact with the tissues of humans and lower animals with undue toxicity, irritation, allergic response, and the like, commensurate with a reasonable benefit/risk ratio, and effective for their intended use, as well as the zwitterionic forms, where possible, of the compounds of the present invention. "Prodrug", as used herein means a compound which is convertible *in vivo* by metabolic means (e.g. by hydrolysis) to afford any compound delineated by the formulae of the instant invention. Various forms of prodrugs are known in the art, for example, as discussed in Bundgaard, (ed.), Design of Prodrugs, Elsevier (1985); Widder, et al. (ed.), Methods in Enzymology, vol. 4, Academic Press (1985); Krogsgaard-Larsen, et al., (ed). "Design and Application of Prodrugs, Textbook of Drug Design and Development, Chapter 5, 113-191 (1991); Bundgaard, et al., Journal of Drug Deliver Reviews, 8:1-38 (1992); Bundgaard, J. of Pharmaceutical Sciences, 77:285 et seq. (1988); Higuchi and Stella (eds.) Prodrugs as Novel Drug Delivery Systems, American Chemical Society (1975); and Bernard Testa & Joachim Mayer, "Hydrolysis In Drug And Prodrug Metabolism: Chemistry, Biochemistry And Enzymology," John Wiley and Sons, Ltd. (2002).

As used herein, the term "pharmaceutically acceptable salt" refers to those salts of the compounds formed by the process of the present invention which are, within the scope of sound medical judgment, suitable for use in contact with the tissues of humans and lower animals without undue toxicity, irritation, allergic response and the like, and are commensurate with a reasonable benefit/risk ratio. Pharmaceutically acceptable salts are well known in the art. For example, S. M. Berge, *et al.* describes pharmaceutically acceptable salts in detail in J. Pharmaceutical Sciences, 66:1-19 (1977). The salts can be prepared *in situ* during the final isolation and purification of the molecules of the invention, or separately by reacting the free base function with a suitable organic acid. Examples of pharmaceutically acceptable include, but are not limited to, nontoxic acid addition salts are salts of an amino group formed with inorganic acids such as hydrochloric acid, hydrobromic acid, phosphoric acid, sulfuric acid and perchloric acid or with organic acids such as acetic acid, maleic acid, tartaric acid, citric acid, succinic acid or malonic acid or by using other methods used in the art such as ion exchange. Other pharmaceutically acceptable salts include, but are not limited to, adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, valerate salts, and the like. Representative alkali or alkaline earth metal salts include sodium, lithium, potassium, calcium, magnesium, and the like. Further pharmaceutically acceptable salts include, when appropriate, nontoxic ammonium, quaternary ammonium, and amine cations formed using counterions such as halide, hydroxide, carboxylate, sulfate, phosphate, nitrate, alkyl having from 1 to 6 carbon atoms, sulfonate and aryl sulfonate.

As used herein, the term "kit" refers to any delivery system for delivering materials. Inclusive of the term "kits" are kits for both research and clinical applications. In the context of reaction assays, such delivery systems include systems that allow for the storage, transport, or delivery of reaction reagents (e.g., oligonucleotides, enzymes, etc. in the appropriate containers) and/or supporting materials (e.g., buffers, written instructions for performing the assay etc.) from one location to another. For example, kits include one or more enclosures (e.g., boxes) containing the relevant reaction reagents and/or supporting materials. As used herein, the term "fragmented kit" refers to delivery systems comprising two or more separate containers that each contain a subportion of the total kit components. The containers may be delivered to the intended recipient together or separately. For example, a first container may contain an enzyme for use in an assay, while a second container contains oligonucleotides. The term "fragmented kit" is intended to encompass kits containing Analyte specific reagents (ASR's) regulated under section 520(e) of the Federal Food, Drug, and Cosmetic Act, but are not limited thereto. Indeed, any delivery system comprising two or more separate containers that each contains a subportion of the total kit components are included in the term "fragmented kit." In contrast, a "combined kit" refers to a delivery system containing all of the components of a reaction assay in a single container (e.g., in a single box housing each of the desired components). The term "kit" includes both fragmented and combined kits.

### General Techniques

For further elaboration of general techniques useful in the practice of this invention, the practitioner can refer to standard textbooks and reviews in cell biology, tissue culture, embryology, and physiology.

With respect to tissue culture and embryonic stem cells, the reader may wish to refer to Teratocarcinomas and embryonic stem cells: A practical approach (E. J. Robertson, ed., IRL Press Ltd. 1987); Guide to Techniques in Mouse Development (P. M. Wasserman et al. eds., Academic Press 1993); Embryonic Stem Cell Differentiation in vitro (M. V. Wiles, Meth. Enzymol. 225:900, 1993); Properties and uses of Embryonic Stem Cells: Prospects for Application to Human Biology and Gene Therapy (P. D. Rathjen et al., Reprod. Fertil. Dev. 10:31, 1998).

General methods in molecular and cellular biochemistry can be found in such standard textbooks as Molecular Cloning: A Laboratory Manual, 3rd Ed. (Sambrook et al., Harbor Laboratory Press 2001); Short Protocols in Molecular Biology, 4th Ed. (Ausubel et al. eds., John Wiley & Sons 1999); Protein Methods (Bollag et al., John Wiley & Sons 1996); Nonviral Vectors for Gene Therapy (Wagner et al. eds., Academic Press 1999); Viral Vectors (Kaplift & Loewy eds., Academic Press 1995); Immunology Methods Manual (I. Lefkovits ed., Academic Press 1997); and Cell and Tissue Culture: Laboratory Procedures in Biotechnology (Doyle & Griffiths, John Wiley & Sons 1998). Reagents, cloning vectors, and kits for genetic manipulation referred to in this disclosure are available from commercial vendors such as BioRad, Stratagene, Invitrogen, Sigma-Aldrich, and ClonTech.

### Compositions

Matrix metalloproteinases (MMPs) are a family of structurally related zinc-dependent proteolytic enzymes that digest extracellular matrix proteins such as collagen, elastin, laminin and fibronectin. At least 22 different mammalian MMP- proteins have been identified and they are grouped based on substrate specificity and domain structure. Enzymatic activities of the MMPs are precisely controlled, not only by their gene expression in various cell types, but also by activation of their inactive zymogen precursors (proMMPs) and inhibition by endogenous inhibitors and tissue inhibitors of metalloproteinases (TIMPs). The enzymes play a key role in normal homeostatic tissue remodeling events, but are also considered to play a key role in pathological destruction of the matrix in many connective tissue diseases such as arthritis, periodontitis, and tissue ulceration and also in cancer cell invasion and metastasis.

Embodiments of the invention are directed to compositions comprising one or more matrix metalloproteinases (MMPs). The MMPs can be in an activated state or in an inactive form, such as for example, a proenzyme form (proMMP), or contain active fragments. The MMPs in the various compositions embodied herein comprise at least one a matrix metalloproteinase (MMP) or proMMPs thereof. In other embodiments, the MMPs in the various compositions embodied herein comprise at least two matrix metalloproteinases (MMPs) or proMMPs thereof. In other embodiments, the MMPs in the various compositions embodied herein comprise at least three matrix metalloproteinases (MMPs) or proMMPs thereof. In other embodiments, the MMPs in the various compositions embodied herein comprise at least four matrix metalloproteinases (MMPs) or proMMPs thereof. In other embodiments, the MMPs in the various compositions embodied herein comprise at least five matrix metalloproteinases (MMPs) or proMMPs thereof. In other embodiments, the MMPs in the various compositions embodied herein comprise at least six, seven, or more matrix metalloproteinases (MMPs) or proMMPs thereof. As discussed above, the terms "MMPs" or proMMPs", unless stated otherwise, in the various compositions embodied herein comprises nucleic acid sequences, amino acid sequences, active fragments, synthetic fragments, mutants, including deletions, substitutions, and truncations, as well as modified forms thereof, derivatives, variants, homologous and/or orthologous genes, cDNA, RNA, chimeric molecules, isoforms, mutant genes, splice variants genes, gene polymorphisms, or any combinations thereof. The molecules embodied herein, includes pharmaceutical compositions thereof, a pharmaceutically acceptable salt or prodrug thereof, or any combinations thereof.

It may be desired to degrade certain tissue components first, for example, collagen. In embodiments, the composition comprises MMPs in both active and inactive forms. For example, one MMP can be in an active form, a second MMP can be in an inactive form. The inactive MMP can be activated at any time by addition of an activating agent, e.g. an enzyme. Thus, one tissue component can be degraded first, followed by another etc.

In a preferred embodiment, a composition comprises an effective amount of a matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof.

In a preferred embodiment, a composition comprises an effective amount of at least two matrix metalloproteinases (MMPs) or proMMPs thereof, wherein the MMPs or proMMPs thereof, comprise: MMP-1, MMP-2, MMP-3, MMP-7, MMP-8, MMP-9, MMP-10, MMP-1 1, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof.

In a preferred embodiment, a composition comprises an effective amount of at least three matrix metalloproteinases (MMPs) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof.

In a preferred embodiment, a composition comprises an effective amount of at least four, at least five, at least six or seven or more matrix metalloproteinases (MMPs) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof.

In preferred embodiments, the MMP, the inactive MMP or a proenzyme (proMMP) comprise: proteins, peptides, polypeptides, nucleic acid sequences, cDNA, ribonucleic acid sequences, chimeric molecules, peptidomimetics, peptide nucleic acids (PNA), or combinations thereof.

In other embodiments, the composition comprises an effective amount of a matrix metalloproteinase (MMP) or proMMPs thereof, wherein the MMPs or proMMPs thereof, comprise: MMP-1, MMP-2, MMP-3, MMP-8, MMP-9, MMP-11, MMP-12, MMP-13, MMP-19, MMP-25, active fragments, mutants, variants, pharmaceutical compositions thereof, a pharmaceutically acceptable salt or prodrug thereof, or any combinations thereof. In other embodiments, the composition comprises an effective amount of at least two, at least three, four, or more, matrix metalloproteinases (MMP) or proMMPs thereof, wherein the MMPs or proMMPs thereof, comprise: MMP-1, MMP-2, MMP-3, MMP-8, MMP-9, MMP-11, MMP-12, MMP-13, MMP-19, MMP-25, active fragments, mutants, variants, pharmaceutical compositions thereof, a pharmaceutically acceptable salt or prodrug thereof, or any combinations thereof.

In a preferred embodiment, a composition comprises an effective amount of a matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof, in a pharmaceutical composition.

In a preferred embodiment, a composition comprises an effective amount of a matrix metalloproteinase (MMP) or proMMPs thereof, wherein the MMPs or proMMPs thereof, comprise: MMP-1, MMP-2, MMP-3, MMP-8, MMP-9, MMP-11, MMP-12, MMP-13, MMP-19, MMP-25, active fragments, mutants, variants or any combinations thereof in a pharmaceutical composition.

In some embodiments, the effective amount of any one MMP or proenzyme (i.e. proMMP) thereof, catabolizes adipose tissue. In other embodiments, an effective amount of any two or more MMPs or proMMPs thereof, dissociate or catabolize adipose tissue.

Adipose tissue extracellular matrix (ECM) has been described as "loose connective tissue." Immunofluorescence staining of bovine adipose tissue ECM revealed types I, III, IV, V, and VI collagen, laminin, and fibronectin. More detailed proteomic analyses have shown some variation based on species, but the collagens are consistent. Decorin or other proteoglycans may be important in adipose tissue ECM. Quantitation of human adipose tissue ECM showed significant levels of acid-soluble collagen and elastin, but only low levels of sulfated glycoaminoglycans (GAGs). The matrix metalloproteinases (MMPs) are a family of enzymes capable of catalyzing the degradation of virtually all ECM components, including collagens, laminin, fibronectin, and elastin. Broad-spectrum inhibition of MMPs impairs adipose tissue growth, while MMP-3 and MMP-11 deficient mice developed more adipose tissue than wild-type mice. This indicates that MMPs participate in adipose tissue remodeling.

Considering the adipose tissue ECM composition, in some embodiments the composition comprises a mixture of MMPs for efficient digestion of the ECM and release of ADSCs. For example, the combination of MMP-1 or MMP-8 (for types I and III collagen), MMP-3 or MMP-19 (for type IV collagen, fibronectin, and laminin), and MMP-2 or MMP-9 (for types IV and V collagen) might be needed. If digestion of type VI collagen is desired, MMP-11 can be used, while elastin digestion would need MMP-12.

Examples of MMPs and their specific substrates include, without limitations: MMP-1, substrates include collagen I, II, III, VII, VIII, X, gelatin. MMP-2, substrates include gelatin, collagen I, II, III, IV, VII, X. MMP-3, substrates include collagen II, IV, IX, X, XI, gelatin. MMP-7, substrates include: fibronectin, laminin, collagen IV, gelatin. MMP-8, substrates include collagen I, II, III, VII, VIII, X, aggrecan, gelatin. MMP-9, substrates include gelatin, collagen IV, V. MMP-10, substrates include collagen IV, laminin, fibronectin, elastin. MMP-11, substrates include collagen IV, fibronectin, laminin, aggrecan. MMP-12, substrates include elastin, fibronectin, collagen IV. MMP-13, substrates include Col I, II, III, IV, IX, X, XIV, gelatin. MMP-14, substrates include gelatin, fibronectin, laminin. MMP-15, substrates include gelatin, fibronectin, laminin. MMP-16, substrates include gelatin, fibronectin, laminin. MMP-17, substrates include fibrinogen, fibrin. MMP-18 also known as collagenase 4, xcol4, Xenopus collagenase. MMP-19, also known as RASI-1, occasionally referred to as stromelysin-4. MMP-20, also known as enamelysin. MMP-21 also known as X-MMP-. MMP-23A (CA-MMP-) membrane-associated type-II transmembrane cysteine array. MMP-23B - membrane-associated type-II transmembrane cysteine array. MMP-24 (MT5-MMP-) membrane-associated type-I transmembrane MMP-. MMP-25 (MT6-MMP-) membrane-associated glycosyl phosphatidylinositol-attached. MMP-26 also known as Matrilysin-2, endometase. MMP-27 also known as MMP-22, C-MMP-. MMP-28 also known as epilysin.

Accordingly, the composition of MMPs used to dissociate cell masses (e.g., tumors), proteins, tissues and organs, or to isolate different cell types from a variety of tissues and organs, can be varied depending on the type of cell mass, proteins, tissue or organ to be dissociated, or dissociation of tissue or organ comprising the desired cells for isolation. For example, a mixture of MMPs for the efficient catabolism of adipose tissue comprises two or more MMPs, such as for example, MMP-1, MMP-2, MMP-3, MMP-8, MMP-9, MMP-11, MMP-12, MMP-19 and MMP-25 are included. In this case, MMP-1 and MMP-8 would be chosen based on their ability to efficiently cleave types I-III collagen. MMP-2 and MMP-9 cleave types IV and V collagen. MMP-3 and MMP-19 have activities towards type IV collagen, fibronectin, and laminin, MMP-12 cleaves elastin efficiently, and MMP-11 cleaves type VI collagen. MMPs are typically activated using serine proteases (trypsin, chymotrypsin), followed by a serine protease inhibitor.

In another embodiment, a composition for isolating islet cells from the pancreas or pancreatic tissue, comprises an effective amount of a matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28 active fragments, mutants, variants, pharmaceutical compositions thereof, or any combinations thereof.

In another preferred embodiment, a method of isolating islet cells from a pancreases or pancreatic tissue, comprising: contacting the biological sample with a composition comprises an effective amount of at least two or more matrix metalloproteinases (MMPs) or inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, or any combinations thereof, wherein the composition catabolizes or dissociates the pancreas or pancreatic tissue, thereby isolating stem cells. Preferably, the MMPs, inactive MMPs or proMMPs thereof, optionally comprise one or more active fragments of one or more MMPs, inactive MMPs or proMMPs comprising the active fragment. In another preferred embodiment, the method further comprises administering one or more agents which activate the inactive MMPs or fragments thereof.

In another embodiment, a composition for isolating cardiomyocytes comprises an effective amount of a matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof. In some embodiments, the composition comprises an effective amount of two or more, three or more, four or more matrix metalloproteinase (MMPs), inactive MMPs or proenzymes (proMMPs) thereof.

In another embodiment, a composition for tissue dissociation comprises an effective amount of a matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof. In some embodiments, the composition comprises an effective amount of two or more, three or more, four or more matrix metalloproteinase (MMPs), inactive MMPs or proenzymes (proMMPs) thereof. As discussed above, the tissue can be any type of tissue and the cells can be any type of cells. For example, fibroblasts, dendritic cells, stem cells, etc.

In another embodiment, a composition for isolating viable follicles from human ovarian tissue, comprises an effective amount of a matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof. In some embodiments, the composition comprises an effective amount of two or more, three or more, four or more matrix metalloproteinase (MMPs), inactive MMPs or proenzymes (proMMPs) thereof.

Due to the non-toxicity of the compositions, the isolated cells, can be used for a variety of procedures for treating patients, e.g. *ex vivo* expansion of the cells and re-infusion of the cells to the subject, transplantation, transplantation of the isolated cells, use of stem cells in regenerative medicine, breaking down of cell or tissue masses, such, as for example, in treating fibrotic diseases, etc.

In other embodiments, a composition comprises one or more active fragments of one or more MMPs or proMMPs thereof, comprising the active fragment, wherein the active fragment catabolizes adipose tissue. In some embodiments, the MMPs are active or inactive or the composition comprises combinations of active and inactive forms of MMPs. In other embodiments, the composition further comprises an activating agent, e.g. an enzyme. In other embodiments, the activating agent is independently administered or can be a component of the composition. In other embodiments, the composition comprises a pharmaceutical excipient.

The compositions embodied herein, comprise various concentrations, ratios, types of one or more MMPs. The MMPs present in a particular composition are varied, both in types, amounts etc., depending on the type of tissue or organ for isolation of a particular cell type or for digesting or dissociating a tissue, tissue and cellular masses, or organs and the like. For example, if a tissue comprises interstitial collagens, a "collagenolytic" MMP- [one that catalyzes the hydrolysis of one or more of the interstitial collagens (types I-III) within their triple-helical domain] would be used. Collagenolytic MMPs include the secreted proteases MMP-1, MMP-2, MMP-8, MMP-9, and MMP-13 and the membrane-bound proteases MT1-MMP- and MT2-MMP-. To digest this particular tissue, and, if desired, isolate particular cells, the composition comprises a combination of MMP-1 and/or MMP-8 (for types I and III collagen), MMP-3 and/or MMP-19 (for type IV collagen, fibronectin, and laminin), and MMP-2 and/or MMP-9 (for types IV and V collagen). If the tissue comprises a type VI collagen, MMP-11 would be a component of the MMP- composition. If elastin digestion is required, then MMP-12 can be used.

In other embodiments, a composition comprises one type of MMP and/or proenzyme MMP thereof. In another embodiment, a composition comprises two types of MMPs and/or proenzyme MMPs thereof. In another preferred embodiment, a composition comprises one or more inactive MMPs. In another embodiment, a composition comprises one or more MMPs or proenzymes thereof, activating agents and inhibitors thereof. In such embodiments, the compositions can be separate, that is the MMPs, proenzymes thereof can be kept separate from a composition comprising one or more activating agents agents (for example, trypsin, chymotrypsin, 4-aminophenylmercuric acetate) or yet another composition comprising inhibitors (for example, tissue inhibitors of metalloproteinases (TIMPs), marimastat) of the MMPs.

For example, MMPs can be activated by proteinases or *in vitro* by chemical agents, such as thiol-modifying agents (4-aminophenylmercuric acetate, HgCl₂, and N-ethylmaleimide), oxidized glutathione, SDS, chaotropic agents, and reactive oxygens. Low pH and heat treatment can also lead to activation. Proteolytic activation of MMPs is stepwise in many cases. The initial proteolytic attack occurs at an exposed loop region between the first and the second helices of the propeptide. The cleavage specificity of the bait region is dictated by the sequence found in each MMP. Once a part of the propeptide is removed, this probably destabilizes the rest of the propeptide, which allows the intermolecular processing by partially activated MMP intermediates or other active MMPs. Thus, the final step in the activation is conducted by an MMP.

Activation of proMMPs by plasmin is a relevant pathway *in vivo.* Plasmin is generated from plasminogen by tissue plasminogen activator bound to fibrin and urokinase plasminogen activator bound to a specific cell surface receptor. Both plasminogen and urokinase plasminogen activator are membrane-associated, thereby creating localized proMMP activation and subsequent ECM turnover. Plasmin has been reported to activate proMMP-1, proMMP-3, proMMP-7, proMMP-9, proMMP-10, and proMMP-13. Activated MMPs can participate in processing other MMPs. The stepwise activation system may have evolved to accommodate finer regulatory mechanisms to control destructive enzymes, inasmuch as TIMPs may interfere with activation by interacting with the intermediate MMP before it is fully activated.

Most proMMPs are secreted from cells and activated extracellularly. For example, proMMP-11 (stromelysin 3) is activated intracellularly by furin. ProMMP-11 possesses a furin recognition sequence, KX(R/K)R, at the C-terminal end of the propeptide. Several other MMPs, including the six MT-MMPs, MMP-23, and epilysin (MMP-28), have a similar basic motif in the propeptide. Because these proteins are most likely secreted as active enzymes, their gene expression and inhibition by endogenous inhibitors would be critical for the regulation of activity.

TIMPs are specific inhibitors that bind MMPs in a 1:1 stoichiometry. Four TIMPs (TIMP-1, TIMP-2, TIMP-3, and TIMP-4) have been identified in vertebrates, and their expression is regulated during development and tissue remodeling. Under pathological conditions associated with unbalanced MMP activities, changes of TIMP levels are considered to be important because they directly affect the level of MMP activity.

Proteins such as plasma α-macroglobulins are general endopeptidase inhibitors that inhibit most proteinases by trapping them within the macroglobulin after proteolysis of the bait region of the inhibitor. MMP-1 reacts with α2-macroglobulin more readily than with TIMP-1 in solution.

In other embodiments, the MMPs or proMMPs thereof, optionally comprise one or more active fragments of one or more MMPs or proMMPs comprising the active fragment, wherein the active fragment catabolizes adipose tissue.

In other embodiments, the MMPs or fragments thereof, are active or inactive or the composition contains combinations of MMPs or fragments thereof. In some embodiments, the composition comprises one or more agents which activate the inactive MMPs or fragments thereof.

*Peptides:* For illustrative purposes only, the term "MMP" will also include the proMMP form, active forms, inactive forms and active fragments thereof. The term includes, without limitation, allelic variants, species variants, splicing variants, mutants, fragments, and the like.

In embodiments, a composition comprises a peptide or protein of: a matrix metalloproteinase (MMP), an inactive MMP or a proenzyme (proMMP) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof. In preferred embodiments, the composition further comprises a pharmaceutically acceptable agent, a pharmaceutically acceptable salt or prodrug thereof.

In some embodiments, the composition comprises a peptide or protein of two or more a matrix metalloproteinases (MMPs), inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, variants, mutants, a pharmaceutically acceptable agent, a pharmaceutically acceptable salt or prodrug thereof, or any combinations thereof.

In some embodiments, the composition comprises a peptide or protein of three or more matrix metalloproteinases (MMPs), inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, variants, mutants, a pharmaceutically acceptable agent, a pharmaceutically acceptable salt or prodrug thereof, or any combinations thereof. In some embodiments, the composition comprises a peptide or protein of four or more matrix metalloproteinases (MMPs), inactive MMPs or proenzymes (proMMPs) thereof. In some embodiments, the composition comprises an effective amount of a peptide or protein of five or more matrix metalloproteinases (MMPs), inactive MMPs or proenzymes (proMMPs) thereof.

In a preferred embodiment, MMP peptides comprise at least five consecutive amino acid residues with the understanding that they are "active" peptides. "Active" includes one or more functions of each MMP which includes known functions as described herein but also any other function that is innate to the MMP molecules or including one which may be altered based on any manipulation by the end user.

In another preferred embodiment, MMP peptide includes the peptide itself, chemical equivalents thereto, isomers thereof (e.g., isomers, stereoisomers, retro isomers, retro-inverso isomers, all-[D] isomers, all-[L] isomers, or mixed [L] and [D] isomers thereof), conservative substitutions therein, precursor forms thereof, endoproteolytically-processed forms thereof, such as cleavage of single amino acids from N or C terminals or active metabolites of the peptides of the invention, pharmaceutically-acceptable salts and esters thereof, and other forms resulting from post-translational modification. Also included is any parent sequence, up to and including 10, 9, 8, 7, 6, 5 and 4 amino acids in length (cyclized, or linear, or branched from the core parent sequence), for which the specified sequence is a subsequence. A person skilled in the art would appreciate that where the peptide can be a monomer, dimer, a trimer, etc. The use of the peptides of the present invention include use of peptides wherein the active fragment or fragments are complexed to one or more binding partners. Modified peptides which retain the activity of the peptides of the invention are encompassed within the scope of the present invention.

In another preferred embodiment, a MMP peptide comprises at least one non-native amino acid residue or a non-amino acid molecule. A "non-native" amino acid residue comprises any change to an amino acid which is encoded by the MMP nucleic acid sequence. Thus, a non-native amino acid residue or non-amino acid molecule comprises, without limitation: a chemical equivalent, analog, synthetic molecule, derivative, variant, substitution, peptide nucleic acid, a linker molecule, inorganic molecule etc.

The mutations can be introduced at the nucleic acid level or at the amino acid level. With respect to particular nucleic acid sequences, because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. If mutations at the nucleic acid level are introduced to encode a particular amino acid, then one or more nucleic acids are altered. For example proline is encoded by CCC, CCA, CCG, CCU; thus, one base change, e.g. CCC (proline) to GCC gives rise to alanine. Thus by way of example every natural or non-natural nucleic acid sequence herein which encodes a natural or non-natural polypeptide also describes every possible silent variation of the natural or non-natural nucleic acid. One of skill will recognize that each codon in a natural or non-natural nucleic acid (except AUG, which is ordinarily the only codon for methionine, and TGG, which is ordinarily the only codon for tryptophan) can be modified to yield a functionally identical molecule or a different molecule. Accordingly, each silent variation of a natural and non-natural nucleic acid which encodes a natural and non-natural polypeptide is implicit in each described sequence.

As to amino acid sequences, individual substitutions, deletions or additions to a nucleic acid, peptide, polypeptide, or protein sequence which alters, adds or deletes a single natural and non-natural amino acid or a small percentage of natural and non-natural amino acids in the encoded sequence, the alteration results in the deletion of an amino acid, addition of an amino acid, or substitution of a natural and non-natural amino acid with a chemically similar amino acid. Conservative substitution tables providing functionally similar natural amino acids are well known in the art. Such conservatively modified variants are in addition to and do not exclude polymorphic variants, interspecies homologs, and alleles of the methods and compositions described herein.

A "non-natural amino acid" refers to an amino acid that is not one of the 20 common amino acids or pyrolysine or selenocysteine. Other terms that may be used synonymously with the term "non-natural amino acid" is "non-naturally encoded amino acid," "unnatural amino acid," "non-naturally-occurring amino acid," and variously hyphenated and non-hyphenated versions thereof. The term "non-natural amino acid" includes, but is not limited to, amino acids which occur naturally by modification of a naturally encoded amino acid (including but not limited to, the 20 common amino acids or pyrrolysine and selenocysteine) but are not themselves incorporated, without user manipulation, into a growing polypeptide chain by the translation complex. Examples of naturally-occurring amino acids that are not naturally-encoded include, but are not limited to, N-acetylglucosaminyl-L-serine, N-acetylglucosaminyl-L-threonine, and O-phosphotyrosine. Additionally, the term "non-natural amino acid" includes, but is not limited to, amino acids which do not occur naturally and may be obtained synthetically or may be obtained by modification of non-natural amino acids.

In some cases, the non-natural amino acid substitution(s) or incorporation(s) will be combined with other additions, substitutions, or deletions within the polypeptide to affect other chemical, physical, pharmacologic and/or biological traits. In some cases, the other additions, substitutions or deletions may increase the stability (including but not limited to, resistance to proteolytic degradation) of the polypeptide or increase affinity of the polypeptide for its appropriate receptor, ligand and/or binding proteins. In some cases, the other additions, substitutions or deletions may increase the solubility of the polypeptide. In some embodiments sites are selected for substitution with a naturally encoded or non-natural amino acid in addition to another site for incorporation of a non-natural amino acid for the purpose of increasing the polypeptide solubility following expression in recombinant host cells. In some embodiments, the polypeptides comprise another addition, substitution, or deletion that modulates affinity for the associated ligand, binding proteins, and/or receptor, modulates (including but not limited to, increases or decreases) receptor dimerization, stabilizes receptor dimers, modulates circulating half-life, modulates release or bio-availability, facilitates purification, or improves or alters a particular route of administration. Similarly, the non-natural amino acid polypeptide can comprise chemical or enzyme cleavage sequences, protease cleavage sequences, reactive groups, antibody-binding domains (including but not limited to, FLAG or poly-His) or other affinity based sequences (including but not limited to, FLAG, poly-His, GST, etc.) or linked molecules (including but not limited to, biotin) that improve detection (including but not limited to, GFP), purification, transport thru tissues or cell membranes, prodrug release or activation, size reduction, or other traits of the polypeptide.

The methods and compositions described herein include incorporation of one or more non-natural amino acids into a polypeptide. One or more non-natural amino acids may be incorporated at one or more particular positions which does not disrupt activity of the polypeptide. This can be achieved by making "conservative" substitutions, including but not limited to, substituting hydrophobic amino acids with non-natural or natural hydrophobic amino acids, bulky amino acids with non-natural or natural bulky amino acids, hydrophilic amino acids with non-natural or natural hydrophilic amino acids) and/or inserting the non-natural amino acid in a location that is not required for activity.

A variety of biochemical and structural approaches can be employed to select the desired sites for substitution with a non-natural amino acid within the polypeptide. Any position of the polypeptide chain is suitable for selection to incorporate a non-natural amino acid, and selection may be based on rational design or by random selection for any or no particular desired purpose. Selection of desired sites may be based on producing a non-natural amino acid polypeptide (which may be further modified or remain unmodified) having any desired property or activity, including but not limited to agonists, super-agonists, partial agonists, inverse agonists, antagonists, receptor binding modulators, receptor activity modulators, modulators of binding to binder partners, binding partner activity modulators, binding partner conformation modulators, dimer or multimer formation, no change to activity or property compared to the native molecule, or manipulating any physical or chemical property of the polypeptide such as solubility, aggregation, or stability. For example, locations in the polypeptide required for biological activity of a polypeptide can be identified using methods including, but not limited to, point mutation analysis, alanine scanning or homolog scanning methods. Residues other than those identified as critical to biological activity by methods including, but not limited to, alanine or homolog scanning mutagenesis may be good candidates for substitution with a non-natural amino acid depending on the desired activity sought for the polypeptide. Alternatively, the sites identified as critical to biological activity may also be good candidates for substitution with a non-natural amino acid, again depending on the desired activity sought for the polypeptide. Another alternative would be to make serial substitutions in each position on the polypeptide chain with a non-natural amino acid and observe the effect on the activities of the polypeptide. Any means, technique, or method for selecting a position for substitution with a non-natural amino acid into any polypeptide is suitable for use in the methods, techniques and compositions described herein.

The structure and activity of naturally-occurring mutants of a polypeptide that contain deletions can also be examined to determine regions of the protein that are likely to be tolerant of substitution with a non-natural amino acid. Once residues that are likely to be intolerant to substitution with non-natural amino acids have been eliminated, the impact of proposed substitutions at each of the remaining positions can be examined using methods including, but not limited to, the three-dimensional structure of the relevant polypeptide, and any associated ligands or binding proteins. X-ray crystallographic and NMR structures of many polypeptides are available in the Protein Data Bank (PDB, rcsb.org), a centralized database containing three-dimensional structural data of large molecules of proteins and nucleic acids, one can be used to identify amino acid positions that can be substituted with non-natural amino acids. In addition, models may be made investigating the secondary and tertiary structure of polypeptides, if three-dimensional structural data is not available. Thus, the identity of amino acid positions that can be substituted with non-natural amino acids can be readily obtained. Exemplary sites of incorporation of a non-natural amino acid include, but are not limited to, those that are excluded from potential receptor binding regions, or regions for binding to binding proteins or ligands may be fully or partially solvent exposed, have minimal or no hydrogen-bonding interactions with nearby residues, may be minimally exposed to nearby reactive residues, and/or may be in regions that are highly flexible as predicted by the three-dimensional crystal structure of a particular polypeptide with its associated receptor, ligand or binding proteins.

A wide variety of non-natural amino acids can be substituted for, or incorporated into, a given position in a polypeptide. By way of example, a particular non-natural amino acid may be selected for incorporation based on an examination of the three dimensional crystal structure of a polypeptide with its associated ligand, receptor and/or binding proteins, a preference for conservative substitutions

As further used herein, a "chemical equivalent" of a peptide of the invention is a molecule which possesses the same desired activity, e.g. collagenase activity, as peptides described herein, and exhibits a trivial chemical different, or a molecule which is converted, under mild conditions, into a peptide of the invention (e.g., esters, ethers, reduction products, and complexes of the peptides of the invention).

Additionally, as used herein, "conservative substitutions" are those amino acid substitutions which are functionally equivalent to the substituted amino acid residue, either because they have similar polarity or steric arrangement, or because they belong to the same class as the substituted residue (e.g., hydrophobic, acidic, or basic). The term "conservative substitutions", as defined herein, includes substitutions having an inconsequential effect on the ability of the peptide of the invention to enhance innate immunity. Examples of conservative substitutions include the substitution of a polar (hydrophilic) residue for another (e.g., arginine/lysine, glutamine/asparagine, or threonine/serine); the substitution of a non-polar (hydrophobic) residue (e.g. isoleucine, leucine, methionine, phenylalanine, tyrosine) for another, the substitution of an acidic residue (e.g., aspartic acid or glutamic acid) for another; or the substitution of a basic residue (e.g., arginine, histidine, lysine or ornithine) for another.

The term "analogue", as used herein, includes any peptide having an amino acid sequence substantially identical to a sequence described herein, in which at least one residue has been conservatively substituted with a functionally-similar residue. An "analogue" includes functional variants and obvious chemical equivalents of an amino acid sequence of an MMP- peptide. As further used herein, the term "functional variant" refers to the activity of a peptide that demonstrates an enzymatic capability, such as, for example, catalyzes the hydrolysis of one or more of the interstitial collagens. An "analogue" further includes any pharmaceutically-acceptable salt of an analogue as described herein.

A "derivative", as used herein, refers to a peptide of the invention having one or more amino acids chemically derivatized by reaction of a functional side group. Exemplary derivatized molecules include, without limitation, peptide molecules in which free amino groups have been derivatized to form salts or amides, by adding acetyl groups, amine hydrochlorides, carbobenzoxy groups, chloroacetyl groups, formyl groups, p-toluene sulfonyl groups, or t-butyloxycarbonyl groups. Free hydroxyl groups may be derivatized to form O-acyl or O-alkyl derivatives. Furthermore, free carboxyl groups may be derivatized to form salts, esters (e.g., methyl and ethyl esters), or hydrazides. Thus, a "derivative" further includes any pharmaceutically-acceptable salt of a derivative as described herein.

In one embodiment of the present invention, the MMP peptides comprise a modified C-terminus and/or a modified N-terminus. For example, the isolated peptide may have an amidated C-terminus. For example, the amino terminus can be acetylated (Ac) or the carboxy terminus can be amidated (NH₂). However, in one embodiment of the invention, the peptides of the invention are preferably not acetylated if such a modification would result in loss of desired activity. Amino terminus modifications include methylating (i.e., --NHCH₃ or --NH(CH₃)₂, acetylating, adding a carbobenzoyl group, or blocking the amino terminus with any blocking group containing a carboxylate functionality defined by RCOO--, where R is selected from the group consisting of naphthyl, acridinyl, steroidyl, and similar groups. Carboxy terminus modifications include replacing the free acid with a carboxamide group or forming a cyclic lactam at the carboxy terminus to introduce structural constraints.

In one embodiment backbone substitutions can be made, such as NH to NCH₃. The peptide may also have a modification (e.g., a point mutation, such as an insertion or a deletion, or a truncation). By way of example, the peptide may comprise an amino acid sequence comprising a modified residue by at least one point insertion of a D amino acid as long as desired activity is retained. For example, proline analogs in which the ring size of the proline residue is changed from 5 members to 4, 6, or 7 members can be employed. Cyclic groups can be saturated or unsaturated, and if unsaturated, can be aromatic or nonaromatic.

In another preferred embodiment, the naturally occurring side chains of the 20 genetically encoded amino acids (or D amino acids) are replaced with other side chains with similar properties, for instance with groups such as alkyl, lower alkyl, cyclic 4-, 5-, 6-, to 7-membered alkyl amide, amide lower alkyl amide di(lower alkyl), lower alkoxy, hydroxy, carboxy and the lower ester derivatives thereof, and with 4-, 5-, 6-, to 7-membered heterocyclic.

Such substitutions can include but are not necessarily limited to: (1) non-standard positively charged amino acids, like: ornithine, Nlys; N-(4-aminobutyl)-glycine which has the lysine side chain attached to the "N-terminus" and compounds with aminopropyl or aminoethyl groups attached to the amino group of glycine; (2) non-naturally occurring amino acids with no net charge and side-chains similar to arginine, such as, Cit; citrulline and Hci; citrulline with one more methylene group; (3) non-standard non-naturally occurring amino acids with OH (e.g., like serine), such as, hSer; homoserine (one more methylene group, Hyp; hydroxyproline, Val(βOH); hydroxyvaline, Pen; penicillamin, (Val(βSH); (4) proline derivatives, such as, D-Pro, such as, 3,4-dehydroproline, Pyr; pyroglutamine, Proline with fluorine substitutions on the ring, 1,3-thiazolidine-+carboxylic acid (proline with S in ring); (5) histidine derivative, such as, Thi; beta-(2-thienyl)-alanine; or (6) alkyl derivatives, such as, Abu; 2-aminobutyric acid (ethyl group on Ca), Nva; norvaline (propyl group on Cα), Nle; norleucine (butyl group on Cα), Hol; homoleucine (propyl group on Cα), Aib, alpha-aminoisobutyric acid (valine without methylene group). A person skilled in the art would appreciate that those substitutions that retain the activity of the parent peptide/sequence.

In another alternative embodiment, the C-terminal carboxyl group or a C-terminal ester can be induced to cyclize by internal displacement of the --OH or the ester (--OR) of the carboxyl group or ester respectively with the N-terminal amino group to form a cyclic peptide. For example, after synthesis and cleavage to give the peptide acid, the free acid is converted to an activated ester by an appropriate carboxyl group activator such as dicyclohexylcarbodiimide (DCC) in solution, for example, in methylene chloride (CH₂Cl₂), dimethyl formamide (DMF) mixtures. The cyclic peptide is then formed by internal displacement of the activated ester with the N-terminal amine. Internal cyclization as opposed to polymerization can be enhanced by use of very dilute solutions. Such methods are well known in the art.

The peptides of the invention can be cyclized, or a desamino or descarboxy residue at the termini of the peptide can be incorporated, so that there is no terminal amino or carboxyl group, for example, to remain inactive until such time an activating agent is administered, or to restrict the conformation of the peptide. C-terminal functional groups of the compounds of the present invention include amide, amide lower alkyl, amide di(lower alkyl), lower alkoxy, hydroxy, and carboxy, and the lower ester derivatives thereof, and the pharmaceutically acceptable salts thereof.

The peptides of the invention can be cyclized by adding an N and/or C terminal cysteine and cyclizing the peptide through disulfide linkages or other side chain interactions.

*Nucleic Acids*: Embodiments of the invention are also directed to nucleic acid sequences, compositions comprising nucleic acids encoding MMPs, proMMPs, active fragments thereof, mutants, variants or combinations thereof. The MMP- nucleic acid sequences can comprise one or more mutations, substitutions, deletions, insertions, modifications, modified nucleobases, linkages, analogs, derivatives and the like. The term "MMP" is meant to be inclusive of all of these molecules. Thus, when referring to MMP-1, the term refers to MMP-1 protein, proMMP-1, active fragments thereof, mutants, variants, MMP-1 nucleic acid sequences comprising one or more mutations, substitutions, deletions, insertions, modifications, modified nucleobases, linkages, analogs, derivatives and the like. The term "nucleic acid sequence" will be used for the sake of brevity and will include, without limitation, isolated nucleic acid or cDNA sequences, synthesized or synthetic nucleic acid sequences, chimeric nucleic acid sequences, homologs, orthologs, variants, mutants or combinations thereof.

In some embodiments, a composition comprises an expression vector having an isolated nucleic acid or cDNA sequence or synthetic nucleic acid sequence, encoding a matrix metalloproteinase (MMP), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof. In some preferred embodiments, the composition comprises an expression vector having an isolated nucleic acid or cDNA sequence or synthetic nucleic acid sequence, encoding two or more, or three or more, or four or more, matrix metalloproteinases (MMPs), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, cDNA sequences, mutants, variants, pharmaceutical compositions thereof, a pharmaceutically acceptable salt or prodrug thereof, or any combinations thereof. In some embodiments, the expression vector encodes a nucleic acid sequence of a matrix metalloproteinase (MMP), an inactive MMPs or a proenzyme (proMMPs) thereof, wherein the MMPs, inactive MMPs or proMMPs thereof, comprise at least about a 50%, 60%, 70%, 75%, 80%, 90%, 95%, 96%, 97%, 99% or 99.9% sequence identity to wild type a matrix metalloproteinase (MMP), an inactive MMPs or a proenzyme (proMMPs) thereof.

In some embodiments, a composition comprises a nucleic acid sequence of a matrix metalloproteinase (MMP), an inactive MMP or a proenzyme (proMMP) thereof, wherein the MMP, inactive MMPor proMMP thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, cDNA sequences, mutants, variants, pharmaceutical compositions thereof, a pharmaceutically acceptable salt or prodrug thereof, or any combinations thereof.

In some embodiments, a composition comprises two or more nucleic acids sequence of a matrix metalloproteinase (MMP), an inactive MMPs or a proenzyme (proMMPs) thereof, wherein the matrix metalloproteinases (MMPs), inactive MMPs or a proenzyme (proMMPs) thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, cDNA sequences, mutants, variants, pharmaceutical compositions thereof, a pharmaceutically acceptable salt or prodrug thereof, or any combinations thereof.

In some embodiments, the composition comprises two or more, three or more, four or more nucleic acid sequences of matrix metalloproteinase (MMPs), inactive MMPs or proenzymes (proMMPs) thereof.

In some embodiments, a composition comprises a nucleic acid sequence of a matrix metalloproteinase (MMP), an inactive MMPs or a proenzyme (proMMPs) thereof, wherein the MMPs, inactive MMPs or proMMPs thereof, comprise at least about a 50% sequence identity to wild type a matrix metalloproteinase (MMP), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, cDNA sequences, mutants, variants, pharmaceutical compositions thereof, a pharmaceutically acceptable salt or prodrug thereof, or any combinations thereof.

In some embodiments, the composition comprises two or more, three or more, four or more nucleic acid sequences of matrix metalloproteinase (MMPs), inactive MMPs or proenzymes (proMMPs), active fragments, cDNA sequences, mutants, variants, pharmaceutical compositions thereof, a pharmaceutically acceptable salt or prodrug thereof, or any combinations thereof.

In other embodiments, an MMP nucleic acid sequence comprises at least about 75%, 80%, 85%, 90%, 95%, 96%, 97%, 98%, 99% sequence identity to wild type MMP or cDNA sequences thereof.

In other embodiments, a matrix metalloproteinase (MMP), an inactive MMPs or a proenzyme (proMMPs) nucleic acid sequences thereof, wherein the MMPs, inactive MMPs or proMMPs nucleic acid sequences thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, or active fragments or cDNA sequences thereof, comprises at least about 75%, 80%, 85%, 90%, 95%, 96%, 97%, 98%, 99% sequence identity to wild type MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments or cDNA sequences thereof.

In some embodiments, a nucleic acid sequence of a MMP further comprises one or more mutations, substitutions, deletions, variants or combinations thereof.

In some embodiments, the homology, sequence identity or complementarity, between a MMP nucleic acid sequence comprising one or more mutations, substitutions, deletions, variants or combinations thereof and the native or wild type or cDNA sequences of a MMP is from about 50% to about 60%. In some embodiments, homology, sequence identity or complementarity, is from about 60% to about 70%. In some embodiments, homology, sequence identity or complementarity, is from about 70% to about 80%. In some embodiments, homology, sequence identity or complementarity, is from about 80% to about 90%. In some embodiments, homology, sequence identity or complementarity, is about 90%, about 92%, about 94%, about 95%, about 96%, about 97%, about 98%, about 99% or about 100%.

In one embodiment, a vector comprises a polynucleotide encoding MMPs or proMMPs thereof, comprising: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, variants, mutants or active fragments thereof.

In one embodiment, the vector expressing one or more MMPs can be administered to a patient wherein expression of MMPs or proMMPs thereof, comprising: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, variants, mutants or active fragments thereof, dissociates proteins or tissues associated with the condition to be treated.

A number of vectors are known to be capable of mediating transfer of gene products to mammalian cells, as is known in the art and described herein. A "vector" (sometimes referred to as gene delivery or gene transfer "vehicle") refers to a macromolecule or complex of molecules comprising a polynucleotide to be delivered to a host cell, either *in vitro* or *in vivo.* The polynucleotide to be delivered may comprise a coding sequence of interest in gene therapy. Vectors include, for example, viral vectors (such as adenoviruses ("Ad"), adeno-associated viruses (AAV), and vesicular stomatitis virus (VSV) and retroviruses), liposomes and other lipid-containing complexes, and other macromolecular complexes capable of mediating delivery of a polynucleotide to a host cell. Vectors can also comprise other components or functionalities that further modulate gene delivery and/or gene expression, or that otherwise provide beneficial properties to the targeted cells. As described and illustrated in more detail below, such other components include, for example, components that influence binding or targeting to cells (including components that mediate cell-type or tissue-specific binding); components that influence uptake of the vector nucleic acid by the cell; components that influence localization of the polynucleotide within the cell after uptake (such as agents mediating nuclear localization); and components that influence expression of the polynucleotide. Such components also might include markers, such as detectable and/or selectable markers that can be used to detect or select for cells that have taken up and are expressing the nucleic acid delivered by the vector. Such components can be provided as a natural feature of the vector (such as the use of certain viral vectors which have components or functionalities mediating binding and uptake), or vectors can be modified to provide such functionalities. Other vectors include those described by Chen et al; BioTechniques, 34: 167-171 (2003). A large variety of such vectors is known in the art and is generally available.

A "recombinant viral vector" refers to a viral vector comprising one or more heterologous gene products or sequences. Since many viral vectors exhibit size-constraints associated with packaging, the heterologous gene products or sequences are typically introduced by replacing one or more portions of the viral genome. Such viruses may become replication-defective, requiring the deleted function(s) to be provided in trans during viral replication and encapsidation (by using, e.g., a helper virus or a packaging cell line carrying gene products necessary for replication and/or encapsidation). Modified viral vectors in which a polynucleotide to be delivered is carried on the outside of the viral particle have also been described (see, e.g., Curiel, D T, et al. PNAS 88: 8850-8854, 1991).

Suitable nucleic acid delivery systems include viral vector, typically sequence from at least one of an adenovirus, adenovirus-associated virus (AAV), helper-dependent adenovirus, retrovirus, or hemagglutinating virus of Japan-liposome (HVJ) complex. Preferably, the viral vector comprises a strong eukaryotic promoter operably linked to the polynucleotide e.g., a cytomegalovirus (CMV) promoter.

Additionally preferred vectors include viral vectors, fusion proteins and chemical conjugates. Retroviral vectors include Moloney murine leukemia viruses and HIV-based viruses. One preferred HIV-based viral vector comprises at least two vectors wherein the *gag* and *pol* genes are from an HIV genome and the *env* gene is from another virus. DNA viral vectors are preferred. These vectors include pox vectors such as orthopox or avipox vectors, herpesvirus vectors such as a herpes simplex I virus (HSV) vector [Geller, A. I. et al., J. Neurochem, 64: 487 (1995); Lim, F., et al., in DNA Cloning: Mammalian Systems, D. Glover, Ed. (Oxford Univ. Press, Oxford England) (1995); Geller, A. I. et al., Proc Natl. Acad. Sci.: U.S.A.: 90 7603 (1993); Geller, A. I., et al, Proc Natl. Acad. Sci. USA: 87:1149 (1990)], Adenovirus Vectors [LeGal LaSalle et al., Science, 259:988 (1993); Davidson, et al., Nat. Genet. 3: 219 (1993); Yang, et al., J. Virol. 69: 2004 (1995)] and Adeno-associated Virus Vectors [Kaplitt, M. G., et al., Nat. Genet. 8:148 (1994)].

Pox viral vectors introduce the gene into the cells cytoplasm. Avipox virus vectors result in only a short term expression of the nucleic acid. Adenovirus vectors, adeno-associated virus vectors and herpes simplex virus (HSV) vectors may be an indication for some invention embodiments. The adenovirus vector results in a shorter term expression (e.g., less than about a month) than adeno-associated virus, in some embodiments, may exhibit much longer expression. The particular vector chosen will depend upon the target cell and the condition being treated. The selection of appropriate promoters can readily be accomplished. Preferably, one would use a high expression promoter. An example of a suitable promoter is the 763-base-pair cytomegalovirus (CMV) promoter. The Rous sarcoma virus (RSV) (Davis, et al., Hum Gene Ther 4:151 (1993)) and MMT promoters may also be used. Certain proteins can be expressed using their native promoter. Other elements that can enhance expression can also be included such as an enhancer or a system that results in high levels of expression such as a tat gene and tar element. This cassette can then be inserted into a vector, e.g., a plasmid vector such as, pUC19, pUC118, pBR322, or other known plasmid vectors, that includes, for example, an *E. coli* origin of replication. See, Sambrook, et al., Molecular Cloning: A Laboratory Manual, Cold Spring Harbor Laboratory press, (1989). The plasmid vector may also include a selectable marker such as the β-lactamase gene for ampicillin resistance, provided that the marker polypeptide does not adversely affect the metabolism of the organism being treated. The cassette can also be bound to a nucleic acid binding moiety in a synthetic delivery system.

If desired, the polynucleotides of the invention may also be used with a microdelivery vehicle such as cationic liposomes and adenoviral vectors.

Replication-defective recombinant adenoviral vectors, can be produced in accordance with known techniques. See, Quantin, et al., Proc. Natl. Acad. Sci. USA, 89:2581-2584 (1992); Stratford-Perricadet, et al., J. Clin. Invest. 90:626-630 (1992); and Rosenfeld, et al., Cell, 68:143-155 (1992).

Expression of the MMPs may be controlled by any promoter/enhancer element known in the art, but these regulatory elements must be functional in the host selected for expression. Promoters which may be used to control MMP- gene expression include, but are not limited to, cytomegalovirus (CMV) promoter (U.S. Pat. Nos. 5,385,839 and 5,168,062), the SV40 early promoter region (Benoist and Chambon, 1981, Nature 290:304-310), the promoter contained in the 3' long terminal repeat of Rous sarcoma virus (Yamamoto, et al., Cell 22:787-797, 1980), the herpes thymidine kinase promoter (Wagner et al., Proc. Natl. Acad. Sci. U.S.A. 78:1441-1445, 1981), the regulatory sequences of the metallothionein gene (Brinster et al., Nature 296:39-42, 1982); prokaryotic expression vectors such as the β-lactamase promoter (VIIIa-Kamaroff, et al., Proc. Natl. Acad. Sci. U.S.A. 75:3727-3731, 1978), or the *tac* promoter (DeBoer, et al., Proc. Natl. Acad. Sci. U.S.A. 80:21-25, 1983); see also promoter elements from yeast or other fungi such as the Gal 4 promoter, the ADC (alcohol dehydrogenase) promoter, PGK (phosphoglycerol kinase) promoter, alkaline phosphatase promoter; and the animal transcriptional control regions, which exhibit tissue specificity and have been utilized in transgenic animals: elastase I gene control region which is active in pancreatic acinar cells (Swift et al., Cell 38:639-646, 1984; Ornitz et al., Cold Spring Harbor Symp. Quant. Biol. 50:399-409, 1986; MacDonald, Hepatology 7:425-515, 1987); insulin gene control region which is active in pancreatic beta cells (Hanahan, Nature 315:115-122, 1985), immunoglobulin gene control region which is active in lymphoid cells (Grosschedl et al., Cell 38:647-658, 1984; Adames et al., Nature 318:533-538, 1985; Alexander et al., Mol. Cell. Biol. 7:1436-1444, 1987), mouse mammary tumor virus control region which is active in testicular, breast, lymphoid and mast cells (Leder et al., Cell 45:485-495, 1986), albumin gene control region which is active in liver (Pinkert et al., Genes and Devel. 1:268-276, 1987), alpha-fetoprotein gene control region which is active in liver (Krumlauf et al., Mol. Cell. Biol. 5:1639-1648, 1985; Hammer et al., Science 235:53-58, 1987), alpha 1-antitrypsin gene control region which is active in the liver (Kelsey et al., Genes and Devel. 1: 161-171, 1987), beta-globin gene control region which is active in myeloid cells (Mogram et al., Nature 315:338-340, 1985; Kollias et al., Cell 46:89-94, 1986), myelin basic protein gene control region which is active in oligodendrocyte cells in the brain (Readhead et al., Cell 48:703-712, 1987), myosin light chain-2 gene control region which is active in skeletal muscle (Sani, Nature 314:283-286, 1985), and gonadotropic releasing hormone gene control region which is active in the hypothalamus (Mason et al., Science 234:1372-1378, 1986).

A wide variety of host/expression vector combinations may be employed in expressing the DNA sequences of this invention. Useful expression vectors, for example, may consist of segments of chromosomal, non-chromosomal and synthetic DNA sequences. Suitable vectors include derivatives of SV40 and known bacterial plasmids, e.g., *E. coli* plasmids col El, pCR1, pBR322, pMal-C2, pET, pGEX (Smith et al., Gene 67:31-40, 1988), pMB9 and their derivatives, plasmids such as RP4; phage DNAs, e.g., the numerous derivatives of phage 1, e.g., NM989, and other phage DNA, e.g., M13 and filamentous single stranded phage DNA; yeast plasmids such as the 2µ plasmid or derivatives thereof, vectors useful in eukaryotic cells, such as vectors useful in insect or mammalian cells; vectors derived from combinations of plasmids and phage DNAs, such as plasmids that have been modified to employ phage DNA or other expression control sequences; and the like.

Yeast expression systems can also be used according to the invention to express MMPs. For example, the non-fusion pYES2 vector (XbaI, SphI, ShoI, NotI, GstXI, EcoRI, BstXI, BamH1, SacI, KpnI, and HindIII cloning sites; Invitrogen) or the fusion pYESHisA, B, C (XbaI, SphI, ShoI, NotI, BstXI, EcoRI, BamH1, SacI, KpnI, and HindIII cloning sites, N-terminal peptide purified with ProBond resin and cleaved with enterokinase; Invitrogen), to mention just two, can be employed according to the invention. A yeast two-hybrid expression system can be prepared in accordance with the invention.

One preferred delivery system is a recombinant viral vector that incorporates one or more of the polynucleotides therein, preferably about one polynucleotide. Preferably, the viral vector used in the invention methods has a pfu (plague forming units) of from about 10⁸ to about 5 × 10¹⁰ pfu. In embodiments in which the polynucleotide is to be administered with a non-viral vector, use of between from about 0.1 nanograms to about 4000 micrograms will often be useful e.g., about 1 nanogram to about 100 micrograms.

### Uses, Formulations, Administration

The composition is useful in a variety of procedures and methods *in vitro* and *in vivo.* For example, isolation of stem cells, isolation of islet cells from pancreas, or isolation of any type of cell from any tissue or organ, degradation of selected tissues, degradation of tissue or cellular masses, degradation of tissue components or tissue scaffolds (e.g. fibrin, collagen, elastin, etc.) cosmetic uses, e.g. catalysis of fat deposits, cellulite and the like.

*Stem Cells:* In one preferred embodiment, a method of isolating islet cells from the pancreas comprises contacting the pancreas with a composition comprising an effective amount of a matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof. The pancreas can be perfused with an MMP cocktail or the pancreas can be sliced prior to incubation with the compositions embodied herein and the islets can be isolated and purified by methods known in the art. In some embodiments, the composition comprises two or more, three or more, four or more matrix metalloproteinases (MMPs), inactive MMPs or proenzymes (proMMPs) thereof.

In another preferred embodiment, a method of dissociating a tissue or organ for isolating various cell types comprises contacting the tissue or organ with a composition comprising an effective amount of a matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof. In some embodiments, the composition comprises two or more, three or more, four or more matrix metalloproteinases (MMPs), inactive MMPs or proenzymes (proMMPs) thereof.

The tissues or organs can be perfused with an MMP cocktail (e.g. combinations of two or more MMP molecules) or the organ can be sliced prior to incubation with the compositions embodied herein and the desired cells can be isolated and purified by methods known in the art. Examples of cell types include without limitations, cardiac myocytes, fibroblast, dendritic cells, follicles from ovarian tissues and the like.

In an embodiment, a method of isolating stem cells from adipose tissue, comprises contacting adipose tissue with a composition comprising an effective amount of at least one matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof, wherein the composition catabolizes the tissue, thereby isolating stem cells from the tissue. In some embodiments, the composition comprises two or more, three or more, four or more matrix metalloproteinases (MMPs), inactive MMPs or proenzymes (proMMPs) thereof.

In an embodiment, a method of isolating stem cells from a biological sample, comprises contacting the biological sample with a composition comprising an effective amount of at least one matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof. For example, the biological sample can be cord blood, bone marrow, bone, placenta, adipose tissue, pancreas, heart, and the like. Examples of stem cells in cord blood: (a) Somatic cells; (b) Mesenchymal stem cells; (c) Endothelial progenitors and angiogenesis stimulating cells; and (d) Hematopoietic stem cells. In some embodiments, a biological sample comprises: an epithelium, connective tissue, adipose tissue, endothelium, basement membranes, basal lamina, cardiac tissues, endocardium, apical membrane, basolateral membrane, extracellular matrix, dense connective tissue, fibrous connective tissue, olfactory epithelium, loose connective tissue, mucins, mesothelium, stroma, reticular connective tissue, bone marrow, blood, blood vessels, lymphatic tissue, lung, cardiovascular tissue, brain tissue, cerebrospinal tissues and fluids, cerebrovascular tissues and fluids, nervous tissue, brain, bone tissue, skin, muscle, pancreatic tissues, ovarian follicles, cord blood tissue, placenta, intestine lining, brain tissue, spinal tissue, cardiovascular tissue, connective tissue, cerebrospinal fluids or tissue, bone marrow, dermis, blood, periosteum, fibrotic tissue, scar tissue, or any organ tissue.

Stem cells or precursor cells that can be isolated from tissues include but are not limited to, e.g., peripheral blood stem cells (PBSC), stem cells isolated from bone marrow (bone marrow cells; BMCs); stem cells isolated from adipose tissue; mesenchymal stem cells (MSCs), stem cells isolated from umbilical cord blood, menstrual fluid, cardiac derived cells, embryonic stem cells, CD30⁺ cells, CD34⁺ cells, CD34⁻ cells, CD9⁺ cells, CD29⁺ cells, CD44⁺ cells, CD45⁺ cells, CD49⁺ cells, CD54⁺ cells, CD56⁺ cells, CD59⁺ cells, CD71⁺ cells, CD90⁺ cells, e.g., CD90.1⁺ or CD90.2⁺ cells, CD105⁺ cells, CD133⁺ cells, CD135⁺ (flt-3⁺) cells, CD140a⁺ cells, CDCP1⁺ cells, CD146⁺ (muc-18⁺) cells, ABCG2⁺ cells, CD144⁺ cells, fetal liver kinase 1⁺ cells, Stro-1⁺ cells, CD117⁺ (c-kit⁺) cells, nestin⁺ cells, PSA-NCAm⁺ cells, CD30⁺ cells, p75neurotophin⁺ cells, CD106⁺ cells, CD120a⁺ cells, CD124⁺ cells, CD166⁺ cells, stem cell factor+ (SCF⁺) cells, Sca-1⁺ cells, SH2⁺ cells, SH3⁺ cells, HLA, e.g., HLA-ABC cells, bone morphogenic protein protein⁺ (BMP) cells, e.g., BMP2⁺ and BMP4⁺ cells, Gap43⁺ cells, glial fibrillary acidic protein⁺ (GFAP⁺) cells, myelin basic protein⁺ (MBP⁺) cells, O4⁺ cells, O1⁺ cells, synaptophysin⁺ cells, alkaline phosphatase⁺ cells, cripto⁺ (TDGF-1⁺) cells, podocalyxin⁺ cells, sulfated proteoglycan⁺ cells, e.g., silylated keratin sulfate proteoglycan⁺ cells, stage-specific embryonic antigen⁺ (e.g., SSEA-1, -3 and -4) cells, TRA-1-60⁺ cells, TRA-1-81⁺ cells, osteocalcin⁺ cells, matrix gla protein⁺ cells, osteopontin⁺ cells, Thy1⁺ cells, collagen type II⁺ cells, collagen type IV⁺ cells, fatty acid transporter⁺ cells, and β1 integrin⁺ cells.

Mesenchymal stem cells (MSCs) are the formative pluripotential blast cells found *inter alia* in bone marrow, blood, dermis and periosteum that are capable of differentiating into more than one specific type of mesenchymal or connective tissue (i.e. the tissues of the body that support the specialized elements; e.g. adipose, osseous, stroma, cartilaginous, elastic and fibrous connective tissues) depending upon various influences from bioactive factors, such as cytokines.

Approximately, 30% of human marrow aspirate cells adhering to plastic are considered as MSCs. These cells can be expanded *in vitro* and then induced to differentiate. The fact that adult MSCs can be expanded *in vitro* and stimulated to form bone, cartilage, tendon, muscle or fat cells render them attractive for tissue engineering and gene therapy strategies. *In vivo* assays have been developed to assay MSC function. MSCs injected into the circulation can integrate into a number of tissues described hereinabove. Specifically, skeletal and cardiac muscle can be induced by exposure to 5-azacytidine and neuronal differentiation of rat and human MSCs in culture can be induced by exposure to β-mercaptoethanol, DMSO or butylated hydroxyanisole [Woodbury (2000) J. Neurosci. Res. 61:364-370]. Furthermore, MSC-derived cells are seen to integrate deep into brain after peripheral injection as well as after direct injection of human MSCs into rat brain; they migrate along pathways used during migration of neural stem cells developmentally, become distributed widely and start to lose markers of HSC specialization [Azizi (1998) Proc. Natl. Acad. Sci. USA 95:3908-3913]. Methods for promoting mesenchymal stem and lineage-specific cell proliferation are disclosed in U.S. Pat. No. 6,248,587.

Epitopes on the surface of the human mesenchymal stem cells (hMSCs) such as SH2, SH3 and SH4 described in U.S. Pat. No. 5,486,359 can be used as reagents to screen and capture mesenchymal stem cell population from a heterogeneous cell population, such as exists, for example, in bone marrow. Precursor mesenchymal stem cells are positive for CD45. These precursor mesenchymal stem cells can differentiate into the various mesenchymal lineages.

In another preferred embodiment, the isolated stem cells are embryonic stem cells, adult stem cells, umbilical cord blood stem cells, somatic stem cells, cancer stem cells, or cardiac stem cells.

Additionally, the stem cells of the current invention may be hematopoietic stem cells, or mesenchymal stem cells. The stem cells of the current invention may be totipotent, pluripotent, multipotent or unipotent stem cells. The stem cells of the current invention may be primary stem cells or may be derived from an established stem cell line, premalignant stem cell line, cancer cell line, or any cell line that manifests any stem cell marker. Primary stem cells may be derived from a cancer patient or a healthy patient.

Preferred stem cells according to this aspect of the present invention are human stem cells.

In some embodiments, the stem cells are isolated from adipose tissue. The stem cells are identified by markers comprising: CD44, CD73, CD90, CD105 or combinations thereof.

*Fat, Fat Deposits, Cellulite:* The composition can be used for cosmetic purposes to reduce fat deposits or in treatment of conditions associated with fat deposits, such as, for example, lipomas. Accumulation of fat stores can occur unevenly in the body. For example, some persons may accumulate fat predominantly in the abdominal cavity while others predominately in the subcutaneous tissue. Gender differences may also be apparent with women accumulating fat in the thighs and lateral buttocks and males in the waist. Women may accumulate fatty deposits of the thighs, which have a rumpled or "peau-de-orange" appearance, resulting in a condition referred to as cellulite. Cellulite may be related to skin architecture which allows subdermal fat herniation, sometimes referred to as adipose papillae. Other factors that may be related to cellulite include altered and/or reduced connective tissue septae, vascular and lymph changes that lead to fluid accumulation, and inflammation. Fat tissue may also accumulate in the form of a fibrous fatty deposit known as a lipoma. Utilization of fat stores may occur unevenly. Persons who have lost substantial weight may still have regional pockets of fat accumulation that are resistant to reduction unless unhealthy extremes of weight loss are achieved. Exercise may affect subcutaneous fat stores differently, with deeper tissues responding with lipolysis and superficial stores being more resistant. Cellulite may also still be present despite weight loss, and lipomas are typically not affected by weight loss.

Provided herein are pharmaceutical compositions, formulations, methods, and systems to achieve regional fat, adipose tissue, cellulite and adipocyte reduction therapy.

In some embodiment, a method of reducing a regional fat deposit in a subject in need thereof (e.g., a subject suffering from obesity), comprising administering to the subject, a pharmaceutical composition comprising an effective amount of at least one matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof, wherein the regional fat deposit is reduced.

In one embodiment, a method of reducing a regional fat deposit in a subject in need thereof (e.g., a subject suffering from obesity), comprising administering to the subject, a pharmaceutical composition comprising an effective amount of at least one matrix metalloproteinase (MMP) or proMMPs thereof, wherein the MMPs or proMMPs thereof, comprise: MMP-1, MMP-2, MMP-3, MMP-8, MMP-9, MMP-11, MMP-12, MMP-13, MMP-19, MMP-25, active fragments, mutants, variants, pharmaceutical compositions thereof, a pharmaceutically acceptable salt or prodrug thereof, or any combinations thereof, wherein the regional fat deposit is reduced. In some embodiments, the composition comprises two or more, three or more, four or more matrix metalloproteinases (MMPs), inactive MMPs or proenzymes (proMMPs) thereof.

In embodiments, the pharmaceutical composition is administered by a parenteral, topical, intramuscular, subcutaneous, or transdermal route of administration. In certain aspects, the pharmaceutical composition is administered at or near the regional fat deposit.

In one embodiment, a method of treating a subject having a condition associated with increased adipose tissue comprising administering to the subject, a composition comprising an effective amount of at least one matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof, wherein the composition catabolizes adipose tissue. In some embodiments, the composition comprises two or more, three or more, four or more matrix metalloproteinases (MMPs), inactive MMPs or proenzymes (proMMPs) thereof.

In embodiments, the MMPs or proMMPs thereof, optionally comprise one or more active fragments of one or more MMPs or proMMPs comprising the active fragment, wherein the active fragment catabolizes adipose tissue. Examples of conditions associated with adipose tissue increase comprise: cellulite, fat deposits, lipomas, obesity, diabetes, metabolic diseases or combinations thereof. Thus, the composition is effective in catabolizing any lipid or fat deposits, either for cosmetic or health reasons.

In some embodiments, the composition is administered locally via sub-cutaneous or intra muscular injections. In some embodiments, one or more MMPs or proMMPs are encapsulated and released over time once injected into the subject.

In some embodiments, a liposuction procedure is performed on a subject to whom has been administered a pharmaceutical composition or sustained release pharmaceutical composition comprising a therapeutically effective amount of at least one compound for catabolizing adipose tissue.

*Tissues, Proteins, Matrices, Organs*: In other embodiments, the compositions embodied herein dissociate proteins, matrices, tissues or organs. In a preferred embodiment, a method for dissociating a tissue, comprises contacting the tissue with a composition comprising an effective amount of a matrix metalloproteinase (MMP), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof. Non-limiting examples of tissues include: an epithelium, connective tissue, adipose tissue, endothelium, basement membranes, basal lamina, cardiac tissues, endocardium, apical membrane, basolateral membrane, extracellular matrix, dense connective tissue, fibrous connective tissue, olfactory epithelium, loose connective tissue, mucins, mesothelium, stroma, reticular connective tissue, bone marrow, blood, blood vessels, lymphatic tissue, lung, cardiovascular tissue, brain tissue, cerebrospinal tissues and fluids, cerebrovascular tissues and fluids, nervous tissue, brain, bone tissue, skin, muscle, pancreatic tissues, ovarian follicles, cord blood tissue, placenta, intestine lining, brain tissue, spinal tissue, cardiovascular tissue, connective tissue, cerebrospinal fluids or tissue, bone marrow, dermis, blood, periosteum, fibrotic tissue, scar tissue, or any organ tissue. Specific sources of tissues, or organs, include, without limitation: adipose/fat, adrenal, bone, brain, cartilage, colon, endothelial, epithelial, eye, heart, intestine, kidney, liver, lung, lymph nodes, mammary, miscellaneous muscle, neural, pancreas, parotid, pituitary, prostate, reproductive, scales, skin, spleen, brain stem, thymus, thyroid/parathyroid, tonsil or tumors.

In another preferred embodiment, a method of dissociating a protein matrix, comprises contacting a protein matrix with a composition comprising an effective amount of a matrix metalloproteinase (MMP), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof. Examples of a protein matrix comprise: collagen, fibronectin, gelatin, laminin, aggregan, elastin, fibrin, fibrinogen, or combinations thereof. The compositions can be applied in many medical or cosmetic fields. For example, reduction or elimination of scarring, tissue masses, wound healing, selective removal of tissues and the like.

In other preferred embodiments, a method of treating or healing a scar or a wound comprisescontacting scar tissue or wound with a composition comprising at least one: a matrix metalloproteinase (MMP), an inactive MMP or a proenzyme (proMMP) thereof, wherein the matrix metalloproteinase (MMPs), inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof. Preferably, the MMP, the inactive MMP or a proenzyme (proMMP) comprise: proteins, peptides, polypeptides, nucleic acid sequences, cDNA, ribonucleic acid sequences, chimeric molecules, peptidomimetics, peptide nucleic acids (PNA), or combinations thereof. Preferably, the composition further comprises a pharmaceutically acceptable agent, a pharmaceutically acceptable salt or prodrug thereof. In other preferred embodiments, the composition comprises an effective amount of any two or more MMPs or proMMPs, active fragments, variants, mutants, or any combinations thereof, dissociates or catabolizes the scar tissue or wound.

In another preferred embodiment, a method of dissociating fibrotic tissue comprises contacting the fibrotic tissue with a composition comprising at least one: a matrix metalloproteinase (MMP), an inactive MMP or a proenzyme (proMMP) thereof, wherein the matrix metalloproteinase (MMPs), inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants or any combinations thereof. Preferably, the MMP, the inactive MMP or a proenzyme (proMMP) comprise: proteins, peptides, polypeptides, nucleic acid sequences, cDNA, ribonucleic acid sequences, chimeric molecules, peptidomimetics, peptide nucleic acids (PNA), or combinations thereof. Preferably, the composition further comprises a pharmaceutically acceptable agent, a pharmaceutically acceptable salt or prodrug thereof. In other preferred embodiments, the composition comprises an effective amount of any two or more MMPs or proMMPs, active fragments, variants, mutants, or any combinations thereof, dissociates or catabolizes the fibrotic tissue.

Accordingly, the compositions are also useful for the topical treatment for burn and ulcer clearing, wound healing, scarring or scarred tissue, treatment of Peyronie's disease, treatment of bone (for example, abnormal bone formation or growth), reformation of abnormal or herniated discs.

*Disease*: The compositions embodied herein, are useful for the *in vivo* treatment or prevention of diseases in which a cocktail of MMPs would be beneficial. These include viral diseases, bacterial infections, parasitic of protozoan infections, cancer, autoimmune diseases, inflammation, transplantation, neurological diseases or disorders, Parkinson's disease, Amyotrophic Lateral Sclerosis (ALS), chronic obstructive pulmonary disease (COPD), multiple sclerosis, Alzheimer's Disease, hepatic diseases or disorders, gastrointestinal diseases or disorders, diabetes, cancer, autoimmunity, immune related diseases or disorders, neurological diseases or disorders, neurodegenerative diseases or disorders, nerve repair and paralysis, neuroendocrine differentiation, inflammatory diseases, muscular diseases or disorders, diseases or disorders associated with infectious organisms, and the like.

In particular examples, the compositions herein can be uses in the treatment of, for example, fibrotic diseases, cancer, diseases associated with protein deposits, e.g. Alzheimer's Disease etc., neoplastic diseases, inflammatory diseases, coronary artery diseases, occlusive cardiovascular diseases, degenerative diseases and infectious diseases, cataracts, abnormal protein deposits, and the like. Some examples of neoplastic diseases may be, but not limited to, cancer, lymphoma, leukemia, and brain tumor. Some examples of inflammatory diseases may be, but not limited to, arthritis, asthma, atherosclerosis, Crohn's disease, colitis, dermatitis, lupus erythematous etc. Some examples of infectious diseases may include, but not limited to, are bacterial, viral, fungal, mycoplasmal, certain genetic diseases and other infections

In one preferred embodiment, a method of treating a patient, suffering from or at risk of developing a fibrotic disease, comprises, administering a therapeutically effective amount of a composition comprising an effective amount of a matrix metalloproteinase (MMP) or proMMPs thereof, wherein the MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof. In some embodiments, the composition comprises two or more, three or more, four or more matrix metalloproteinases (MMPs), inactive MMPs or proenzymes (proMMPs) thereof.

"Therapeutically effective amount" means the amount of a compound that, when administered to a subject for treating a disease, is sufficient to effect such treatment for the disease. The "therapeutically effective amount" can vary depending on the compound, the disease and its severity, and the age, weight, etc., of the subject to be treated. The phrase "pharmaceutically acceptable" refers to molecular entities and compositions that are physiologically tolerable and do not typically produce an allergic or similar untoward reaction, such as gastric upset, dizziness and the like, when administered to a human.

The types and amounts of MMPs for use as therapeutic compounds, may be believed to have therapeutic activity on the basis of any information available to the artisan. For example, a prototype compound may be believed to have therapeutic activity on the basis of information contained in the Physician's Desk Reference. In addition, by way of non-limiting example, a therapeutic compound may be believed to have therapeutic activity on the basis of experience of a clinician, structure of the compound, structural activity relationship data, EC₅₀, assay data, IC₅₀ assay data, animal or clinical studies, or any other basis, or combination of such bases.

A therapeutically-active compound is a compound that has therapeutic activity, including for example, the ability of a compound to induce a specified response when administered to a subject or tested *in vitro.* Therapeutic activity includes treatment of a disease or condition, including both prophylactic and ameliorative treatment. Treatment of a disease or condition can include improvement of a disease or condition by any amount, including prevention, amelioration, and elimination of the disease or condition. Therapeutic activity may be conducted against any disease or condition, including in a preferred embodiment against any disease or disorder that would benefit from dissociation of a tissue or mass of cells, for example. In order to determine therapeutic activity any method by which therapeutic activity of a compound may be evaluated can be used. For example, both *in vivo* and *in vitro* methods can be used, including for example, clinical evaluation, EC₅₀, and IC₅₀ assays, and dose response curves.

*Formulations, Administration*: The compositions embodied herein, are formulated for administration by any suitable method, for example, as described in Remington: The Science And Practice Of Pharmacy (21st ed., Lippincott Williams & Wilkins). Exemplary routes of administration include, but are not limited to parenteral, oral, subcutaneous, topical, intramuscular, transdermal, transmucosal, sublingual, intranasal, transvascular, subcutaneous, orbital, or combinations thereof. In some embodiments, the composition is formulated for injection of an area at which treatment is desired, for example, in a regional fat deposit. In another embodiment, the compositions can be formulated as a topical formulation, for example, to eliminate or reduce scarring in a subject. In some embodiments, a composition comprises at least one or more, or at least two or more, or at least three or more matrix metalloproteinases (MMPs), an inactive MMPs or a proenzyme (proMMPs) thereof, wherein the matrix metalloproteinases (MMPs), inactive MMPs or a proenzyme (proMMPs) thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, cDNA sequences, mutants, variants, pharmaceutical compositions thereof, a pharmaceutically acceptable salt or prodrug thereof, or any combinations thereof.

In some embodiments, the composition comprises four or more, or five or more matrix metalloproteinases (MMPs), inactive MMPs or proenzymes (proMMPs) thereof.

The nucleic acids, proteins, peptides, or agents of the present invention may be administered to a patient in need of treatment via any suitable route, including by intravenous, intraperitoneal, intramuscular injection, or orally. The precise dose will depend upon a number of factors, including whether the nucleic acids, proteins, peptides, or agents are for diagnosis or for treatment or for prevention. The dosage or dosing regime of an adult patient may be proportionally adjusted for children and infants, and also adjusted for other administration or other formats, in proportion for example to molecular weight. Administration or treatments may be repeated at appropriate intervals, at the discretion of the physician.

The nucleic acids, proteins, peptides, or agents of the present invention will usually be administered in the form of a pharmaceutical composition, which may comprise at least one component in addition to the nucleic acids, proteins, peptides, or agents. Thus pharmaceutical compositions according to the present invention, and for use in accordance with the present invention, may comprise, in addition to active ingredient, a pharmaceutically acceptable excipient, carrier, buffer, stabilizer or other materials well known to those skilled in the art. Such materials should be non-toxic and should not interfere with the efficacy of the active ingredient. The precise nature of the carrier or other material will depend on the route of administration, which may be oral, or by injection, e.g. intravenous, or by deposition at a tumor site.

In some embodiments, MMPs, proMMPs, activating agents, inhibitors of MMPs or combinations thereof, are formulated as crystalline microparticle suspensions to prolong release and thereby further sustain catabolization of tissues, protein matrices, organs, and the like. The compositions can be administered by topical application, intravenous drip or injection, subcutaneous, intramuscular, intraperitoneal, intracranial and spinal injection, ingestion via oral route, inhalation, trans-epithelial diffusion or an implantable, time-release drug delivery device.

In other embodiments, the compositions embodied herein can be formulated with a carrier, configured for the time release of the MMPs, proMMPs, activating agents, inhibitors of MMPs or combinations thereof. Various time release permutations are contemplated. For example, it may be beneficial to have a sequential release of MMPs in the composition, such that there is a successive or timed release of particular MMPs. This would be particularly desirable in application to tissues, matrices, organs where there are different layers of substrates. For example, if the first layer is collagen and the second layer is fibronectin, it may be desirable to release MMP-2 to degrade the collagen, followed by release of MMP12 to catabolize the fibronectin. Further, if the inactive forms of MMPs are used, it may be desirable to release the MMPs and specific activating agents concomitantly, one prior to the other, one after the other or combinations thereof. Further, if it is desired to include inhibitors specific for an MMP, these can be formulated to release at a point in time after the MMP has catabolized the desired tissue or protein matrix.

In another aspect, the invention provides a pharmaceutical composition comprising an MMP, proMMP, activating agents, inhibitors of MMPs or combinations thereof, or a pharmaceutically acceptable ester, salt, or prodrug thereof, together with a pharmaceutically acceptable carrier. Compositions embodied herein, can be administered as pharmaceutical compositions by any conventional route, in particular enterally, e.g., orally, e.g., in the form of tablets or capsules, or parenterally, e.g., in the form of injectable solutions or suspensions, topically, e.g., in the form of lotions, gels, ointments or creams, or in a nasal or suppository form. Pharmaceutical compositions comprising MMPs, proMMPs, activating agents, inhibitors of MMPs or combinations thereof, of the present invention in free form or in a pharmaceutically acceptable salt form in association with at least one pharmaceutically acceptable carrier or diluent can be manufactured in a conventional manner by mixing, granulating or coating methods. For example, oral compositions can be tablets or gelatin capsules comprising the active ingredient together with a) diluents, e.g., lactose, dextrose, sucrose, mannitol, sorbitol, cellulose and/or glycine; b) lubricants, e.g., silica, talcum, stearic acid, its magnesium or calcium salt and/or polyethyleneglycol; for tablets also c) binders, e.g., magnesium aluminum silicate, starch paste, gelatin, tragacanth, methylcellulose, sodium carboxymethylcellulose and or polyvinylpyrrolidone; if desired d) disintegrants, e.g., starches, agar, alginic acid or its sodium salt, or effervescent mixtures; and/or e) absorbents, colorants, flavors and sweeteners. Injectable compositions can be aqueous isotonic solutions or suspensions, and suppositories can be prepared from fatty emulsions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Suitable formulations for transdermal applications include an effective amount of a compound of the present invention with a carrier. A carrier can include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device to the skin. Matrix transdermal formulations may also be used. Suitable formulations for topical application, e.g., to the skin and eyes, are preferably aqueous solutions, ointments, creams or gels well-known in the art. Such may contain solubilizers, stabilizers, tonicity enhancing agents, buffers and preservatives. Any suitable pharmaceutically acceptable excipient appropriate for a particular route of administration can be used. Examples of pharmaceutically acceptable carriers include, but are not limited to, buffers, saline, or other aqueous media. The compounds of the invention are preferably soluble in the carrier which is employed for their administration (e.g., subcutaneous). Alternatively, a suspension of the active compound or compounds (e.g., a suspension of crystalline microparticles) in a suitable carrier is employed. In some embodiments, one or more of the MMPs or pro MMPs are formulated in a liquid carrier, for example, as a solution, a suspension, a gel, and/or an emulsion. Some embodiments comprise any suitable lipophilic carrier, for example, modified oils (e.g., CREMOPHOR™ BASF, Germany), soybean oil, propylene glycol, polyethylene glycol, derivatized polyethers, combinations thereof, and the like. Some embodiments comprise a microparticulate and/or nanoparticulate carrier for at least one of the MMPs or proMMPs. Some embodiments comprise one or more time released agents, sustained or controlled release carriers or agents, for example, polymer microspheres. Examples of time released agents comprise: glycerol, glycol, erythritol, arabitol, xylitol, mannitol, sorbitol, isomalt, maltitol, lactitol, and polyvinyl alcohol, monosaccharides and disaccharides. The MMPs, etc., can be encapsulated so as to be released over time. Some embodiments comprise excipients suitable for stable suspensions for micronized particles of the MMPs, proMMPs or active fragments thereof, activating agents or inhibitors thereof.

The compositions embodied herein, can be formulated with a carrier material adapted to exhibit a combination of physical characteristics such as biocompatibility, and, preferably, biodegradability and bioabsorbability, while providing a delivery vehicle for release of one or more MMPs, proMMPs, activating agents, inhibitors of MMPs or combinations thereof. The carrier material used is biocompatible such that it results in no induction of inflammation or irritation when implanted, degraded or absorbed.

Thus, the carrier according to the present invention may be either biodegradable or non-biodegradable. Representative examples of biodegradable compositions include albumin, hyaluronic acid, starch, cellulose and cellulose derivatives (e.g., methylcellulose, hydroxypropylcellulose, hydroxypropyl-methylcellulose, carboxymethylcellulose, cellulose acetate phthalate, cellulose acetate succinate, hydroxypropylmethylcellulose phthalate), casein, dextran, polysaccharides, fibrinogen, poly(D,L-lactide), poly(D,L-lactide-co-glycolide), poly(glycolide), poly(hydroxybutyrate), poly(alkylcarbonate) and poly(orthoesters), polyesters, poly(hydroxyvaleric acid), polydioxanone, poly(ethylene terephthalate), poly(malic acid), poly(tartronic acid), polyanhydrides, polyphosphazenes, poly(amino acids) and their copolymers.

Representative examples of non-degradable polymers include poly(ethylene-vinyl acetate) ("EVA") copolymers, silicone rubber, acrylic polymers (polyacrylic acid, polymethylacrylic acid, polymethylmethacrylate, polyalkylcynoacrylate), polyethylene, polypropylene, polyamides (nylon 6,6), polyurethane, poly(ester urethanes), poly(ether urethanes), poly(ester-urea), polyethers (poly(ethylene oxide), poly(propylene oxide), pluronics and poly(tetramethylene glycol)), silicone rubbers and vinyl polymers (polyvinylpyrrolidone, poly(vinyl alcohol), poly(vinyl acetate phthalate). Polymers also may be developed which are either anionic (e.g., alginate, carboxymethyl cellulose and poly(acrylic acid), or cationic (e.g., chitosan, poly-L-lysine, polyethylenimine, and poly(allyl amine)).

Polymeric carriers include poly(ethylene-vinyl acetate), polyurethanes, poly(D,L-lactic acid) oligomers and polymers, poly(L-lactic acid) oligomers and polymers, poly (glycolic acid), copolymers of lactic acid and glycolic acid, poly (caprolactone), poly (valerolactone), polyanhydrides, copolymers of poly (caprolactone) or poly (lactic acid) with a polyethylene glycol (e.g., MePEG), and blends, admixtures, or co-polymers of any of the above. Other preferred polymers include polysaccharides such as hyaluronic acid, chitosan and fucans, and copolymers of polysaccharides with degradable polymers.

Other polymers useful for these applications include carboxylic polymers, polyacetates, polyacrylamides, polycarbonates, polyethers, polyesters, polyethylenes, polyvinylbutyrals, polysilanes, polyureas, polyurethanes, polyoxides, polystyrenes, polysulfides, polysulfones, polysulfonides, polyvinylhalides, pyrrolidones, thermal-setting polymers, cross-linkable acrylic and methacrylic polymers, ethylene acrylic acid copolymers, styrene acrylic copolymers, vinyl acetate polymers and copolymers, vinyl acetal polymers and copolymers, epoxy, melamine, other amino resins, phenolic polymers, and copolymers thereof, water-insoluble cellulose ester polymers (including cellulose acetate propionate, cellulose acetate, cellulose acetate butyrate, cellulose nitrate, cellulose acetate phthalate, and mixtures thereof), polyvinylpyrrolidone, polyethylene glycols, polyethylene oxide, polyvinyl alcohol, polyethers, polysaccharides, hydrophilic polyurethane, polyhydroxyacrylate, dextran, xanthan, hydroxypropyl cellulose, methyl cellulose, and homopolymers and copolymers of N-vinylpyrrolidone, N-vinyllactam, N-vinyl butyrolactam, N-vinyl caprolactam, other vinyl compounds having polar pendant groups, acrylate and methacrylate having hydrophilic esterifying groups, hydroxyacrylate, and acrylic acid, and combinations thereof, cellulose esters and ethers, ethyl cellulose, hydroxyethyl cellulose, cellulose nitrate, cellulose acetate, cellulose acetate butyrate, cellulose acetate propionate, polyurethane, polyacrylate, natural and synthetic elastomers, rubber, acetal, nylon, polyester, styrene polybutadiene, acrylic resin, polyvinylidene chloride, polycarbonate, homopolymers and copolymers of vinyl compounds, polyvinylchloride, polyvinylchloride acetate. In general, see U.S. Pat. No. 6,514,515 to Williams; U.S. Pat. No. 6,506,410 to Park, et al.; U.S. Pat, No. 6,531,154 to Mathiowitz, et al, U.S. Pat. No. 6,344,035 to Chudzik, et al,; U.S. Pat. No. 6,376,742 to Zdrahala, et al.; and Griffith, L. A., Ann. N.Y. Acad. of Sciences, 961:83-95 (2002); and Chaikof, et al, Ann. N.Y. Acad. of Sciences, 961:96-105 (2002).

Additionally, polymers as described herein can also be blended or copolymerized in various compositions as required. The polymeric carriers as discussed can be fashioned in a variety of forms with desired release characteristics and/or with specific desired properties. For example, the polymeric coatings may be fashioned to release the MMPs upon exposure to a specific triggering event such as pH. Representative examples of pH-sensitive polymers include poly(acrylic acid) and its derivatives (including for example, homopolymers such as poly(aminocarboxylic acid); poly(acrylic acid); poly(methyl acrylic acid), copolymers of such homopolymers, and copolymers of poly(acrylic acid) and acrylmonomers such as those discussed above. Other pH sensitive polymers include polysaccharides such as cellulose acetate phthalate; hydroxypropylmethylcellulose phthalate; hydroxypropyl methylcellulose acetate succinate; cellulose acetate trimellilate; and chitosan. Yet other pH sensitive polymers include any mixture of a pH sensitive polymer and a water-soluble polymer.

Likewise, polymeric carriers can be fashioned that are temperature sensitive. Representative examples of thermogelling polymers and their gelatin temperature include homopolymers such as poly(N-methyl-N-n-propylacrylamide) (19.8°C.); poly(N-n-propylacrylamide) (21.5° C.); poly(N-methyl-N-isopropylacrylamide) (22.3° C.); poly(N-n-propylmethacrylamide (28.0° C.); poly(N-isopropylacrylamide) (30.9° C.); poly(N,n-diethylacrylamide) (32.0° C.); poly(N-isopropylmethacrylamide) (44.0° C.); poly(N-cyclopropylacryl-amide) (45.5° C.); poly(N-ethylmethyacrylamide) (50.0° C.); poly(N-methyl-N-ethylacrylamide) (56.0° C.); poly(N-cyclopropylmethacrylamide) (59.0° C.); poly(N-ethylacrylamide) (72.0° C.). Moreover, thermogelling polymers may be made by preparing copolymers between (among) monomers of the above, or by combining such homopolymers with other water-soluble polymers such as acrylmonomers (e.g., acrylic acid and derivatives thereof such as methylacrylic acid, acrylate and derivatives thereof such as butyl methacrylate, acrylamide, and N-n-butyl acrylamide).

Injectable formulations are administered using any method known in the art, for example, using a single needle, multiple needles, and/or using a needleless injection device. In some embodiments, a tissue loading dose of the active ingredients formulated in a suitable carrier delivered by injection. In some embodiments, delivery comprises single needle injection. In some embodiments, delivery comprises injection using a multineedle array, which, in some embodiments, provides a wide dispersion of the formulation in the target tissue. In some embodiments, formulations are injected in a manner that allows dispersal into the appropriate layer of tissue or subcutaneous fat in areas with regional fat.

In some embodiments, the MMPs, the proMMPs, activating agents or any combination thereof, for example injected, as separate formulations, or, alternatively, are administered by separate routes.

In some embodiments a formulation comprises one or more sustained or controlled release agents for providing a sustained or controlled release of MMPs, or proMMPs, or activating agents or active fragments or any combinations thereof. In such formulations, the MMPs or proMMPs, or activating agents or active fragments or any combinations thereof, or are encapsulated in, bound to, and/or conjugated to a sustained or controlled release agent or carrier. In some embodiments, biocompatible, biodegradable sustained or controlled release formulations provide local tissue activity. Sustained release can be over a period from about 12 hours to about 12 months, e.g., one day, 3 days, 7 days, 10 days, 1 month, 45 days, 2 months, 3 months, 4 months, 6 months, 8 months, 9 months, 10 months, 11 months, or any other time period from about 12 hours to about 12 months. Suitable sustained or controlled release agents or carriers include polymers, macromolecules, active ingredient conjugates, hydrogels, contaminations thereof, and the like. Some embodiments of the sustained release carrier target fat, for example, liposomes. Preferably, the sustained release materials are selected to facilitate delivery of a substantially equal amount of the active substance per unit time. Several rounds of injections of the sustained release formulation can be made over time to treat a single area. In some embodiments, sustained release results from formulating the MMPs, etc., as a suspension of crystalline drug microparticles.

In some embodiments, the sustained release agent comprises a polymer, for example, polylactides, polyglycolides, poly(lactide glycolides) polylactic acids, polyglycolic acids, polyanhydrides, polyorthoesters, polyetheresters, polycaprolactones, polyesteramides, polycarbonates, polycyanoacrylates, polyurethanes, polyacrylates, and blends, mixtures, or copolymers of the above, which are used to encapsulate, binds, or conjugate with the active ingredients(s). Some embodiments of sustained release polymers comprise polyethylene glycol groups to which one or more of the active ingredients is conjugated. In some embodiments, the sustained release agent comprises poly(lactide glycolide) (PLGA, poly(lactic-co-glycolic acid)) copolymer.

Some embodiments of the sustained release agent comprise one or more hydrogels, including modified alginates. Examples of suitable modified alginates include those disclosed in WO 98/12228. Some embodiments of the sustained release agent comprise an albumin-based nano-particle carrier or excipient.

In some embodiments, a formulation comprising a prepolymer solution is injected into the target tissue site, where it is then polymerized (e.g., by photopolymerization) or solidified (e.g., by using temperature sensitive gelling materials) *in vivo.*

In some embodiments, the controlled release materials have release characteristics designed for the particular application of tissue, protein or matrix reduction. In some embodiments, the sustained release or controlled release agent is formed into microparticles, such as microspheres, which are formulated as an injectable solution and/or gel. In some embodiments, the microparticles range in size from about 10 µm to about 100 µm in diameter (e.g., about 15 µm, 20 µm, 25 µm, 30 µm, 40 µm, 50 µm, 60 µm, 70 µm, 80 µm, 90 µm or any other diameter from about 10 µm to about 100 µm). In some embodiments, the microparticles are uniform in size. In other embodiments, the microparticles vary in size by about 10% to about 300%, e.g., 30%, 40%, 50%, 70%, 80%, 90%, 120%, 150%, 170%, 190%, 200%, 225%, 250%, 275%, or by any other percentage variation in size from about 10% to about 300%. In some embodiments, formulations comprising MMPs etc., are provided as an injectable gel or processed into microspheres. In other embodiments, they are formed as crystalline microparticles. Other examples of suitable injectable biodegradable, biocompatible materials suitable for microparticle formation include chitosan, dextran, hydroxyapatite, and silicon.

Microspheres and/or microparticles are formed using any method, including by solvent evaporation and/or emulsion polymerization. In some embodiments, the microspheres have average diameters of from about 5 µm to about 60 µm, preferably, about 20 µm. In some embodiments, PLGA is manufactured with varying ratios of lactide to glycolide depending on the desired rate of release of the active ingredient(s). Because the rate of degradation of this copolymer is proportional to its crystallinity and the proportion of glycolide in the formulation, non-racemic mixtures of the lactide and/or glycolide increase crystallinity and slow the rate of degradation. Higher proportions of glycolide increase the rate of degradation. In some embodiments, a ratio of about 65%-75% lactide to about 25%-35% glycolide provides active ingredients released over from about 2 weeks to about 45 days. In other embodiments, the ratio of lactide to glycolide is from about 0:100 to about 100:0, thereby providing other release rates.

Some embodiments of the microspheres or microparticles comprise hollow and/or porous interiors. In some embodiments, the microspheres comprise a solid or porous outer shell.

In some embodiments, formulations comprising a porous outer shell and/or microsphere exhibit a biphasic release profile of the active ingredient(s) with an initial release burst of the active ingredient(s), followed by a sustained release associated with degradation of the polymeric microspheres. While not wishing to be bound by theory, it is thought that the initial release burst loads the tissue with an effective lipolytic or catabolic concentration of the active ingredient(s), with the subsequent slower release maintaining the desired concentration.

In some embodiments, one or more of the active ingredients are encapsulated, bound, and/or conjugated to the polymer at a ratio of about 10-12% by mass compared to the polymer microspheres. The amount of active ingredient as a mass percentage of the carrier (e.g., microparticles or microspheres) is referred to herein as "active ingredient loading." As used herein, the terms "loaded" and "loading" refer to active ingredients substantially encapsulated bound, and/or conjugated to a carrier. In some embodiments, the active ingredient loading is up to about 75%. Thus, some preferred formulations comprise one or more active ingredients, and/or their physiologically acceptable salts and solvates, loaded on polymer microspheres at about 1 mg to about 20 mg of active ingredient (e.g., about 2 mg, 5 mg, 7 mg, 10 mg, 12 mg, 14 mg, 15 mg, 17 mg, 18 mg, or any other amount of active ingredient from about 1 mg to about 20 mg) per about 10 mg to about 200 milligrams of polymer. In some embodiments, a formulation with this active ingredient loading is sufficient for providing from about 12 hours to about 45 days (e.g., about 3 days, 7 days, 16 days, 20 days, 25 days, 30 days, 35 days, 40 days, 42 days, or any other period from about 12 hours to about 45 days) of active ingredient release at a concentration suitable to produce lipolysis as embodied herein.

In some embodiments, two or more active ingredients are loaded into the same microparticle, for example, in a liposome or PLGA. Thus, some embodiments, a polymer encapsulating one or more MMPs, etc is delivered simultaneously to the adipose tissue. Alternatively, the two active ingredients are loaded on separate microparticles. The two types of microspheres are then mixed to obtain a formulation with the desired ratio of each and then administered simultaneously. Alternatively, the two types of microparticles are administered sequentially.

The microspheres comprising the active ingredient(s) are suspended in about 10 ml to about 20 ml of an appropriate physiologically acceptable liquid carrier. In some embodiments using separate microspheres of the active ingredients, the microspheres are mixed together in the liquid carrier. In other embodiments, each type of microspheres is separately mixed with a liquid carrier. In some embodiments, the microsphere suspension is then injected subcutaneously just below the dermis in 1.0 ml aliquots to cover about 2.0 cm² area per ml of the microsphere suspension, for example, for the treatment of cellulite. In some embodiments, about 10 to about 20 injections are administered to cover an area of from about 20 cm² to about 40 cm². Larger and/or smaller areas are treated in various embodiments. Alternatively, in some embodiments, bolus injections of 1.0 ml to 10.0 ml are injected into fat accumulations, such as the submental regions, lateral hips, and buttocks, or tissues. Alternatively, injections as described above are made separately and sequentially in the same locations using two microsphere formulations encapsulating each active ingredient.

In some embodiments, needless injection is used to administer the microparticulate formulations as suspensions or as powdered loaded microparticles, i.e., without a liquid carrier.

PLGA 15 microspheres encapsulate hydrophobic compounds more readily than hydrophilic compounds. To increase loading of hydrophilic active ingredients, in some embodiments, the microspheres are modified with polyethylene glycol units. Microspheres of certain sizes are substantially not absorbed into the blood or removed by lymph, thereby providing localized release of the active ingredient(s) within a target region. For example, in some embodiments, the microspheres are about 20 µm to about 200 µm in diameter, e.g., about 30 µm to about 175 µm, about 50 µm to about 150 µm, about 75 µm to about 125 µm, or any other diameter from about 20 µm to about 200 µm. The size of the microsphere also affects the release profile of the active ingredient(s) in the tissue. In general, larger microspheres provide a longer and more uniform release profile. Accordingly, in some embodiments, the average particle size in the formulation will be selected based on the desired release duration.

In some embodiments, the subject to be treated is provided a non-sustained release formulation. In some embodiments, the non-sustained release formulation, after a single dose, provides activity of one or more MMPs, etc.

In some embodiments, formulations are delivered transdermally using any suitable method known in the art, for example, as a topically applied cream or through a patch. Alternatively, other transdermal delivery means known in the art are also useful, for example, electrical. Sustained release embodiments of transdermally deliverable formulations are also provided, for example, using a biodegradable, biocompatible active ingredient-polymer formulation or liposome formulation, as described herein.

In some embodiments, topical application of the drugs or drug combinations is utilized. For an individual substance the partition coefficient is generally measured as the Octanol:Water ratio or "Log P," and is a measure of a given substance's relative affinity for Octanol vs. Water. The higher the Log P, the more a substance tends to be attracted to Octanol and *vice versa.* In other words, it provides a relative measure of lipophilicity versus hydrophilicity for a given substance. For delivery of agents into the skin an optimal Log P ranges from 1.0 to 5.0. Formoterol has a Log P in the range of 2-4. Ketotifen has similar physical properties that allow it to be delivered into and across the skin.

A variety of topical formulations, including ointments and creams, are suitable for delivery of the drugs or drug combinations. Exemplary topical vehicles for the proposed combinations include, but are not limited to, terpenes (e.g. cineole, linalyl acetate, menthanone, d/l-menthol), fatty acid esters (e.g. isopropyl myristate, ethyl oleate, isopropyl palmitate, butyl myristate), and longer chain alcohols (1-octanol, 1-decanol, 1-dodecanol). N-methyl-pyrrolidone combined with terpenes, fatty acid esters, and longer chain alcohols. Ratios of terpenes, fatty acid esters, and longer chain alcohols to N-methyl-pyrrolidone may be from 100:0 up to a maximum 60:40 weight to weight. In some embodiments, needless intradermal injection of the drugs or drug combinations is used for treatment of wrinkles, scarring and other dermal conditions.

The present invention encompasses pharmaceutically acceptable topical formulations of inventive compounds. The term "pharmaceutically acceptable topical formulation," as used herein, means any formulation which is pharmaceutically acceptable for intradermal administration of a compound of the invention by application of the formulation to the epidermis. In certain embodiments of the invention, the topical formulation comprises a carrier system. Pharmaceutically effective carriers include, but are not limited to, solvents {e.g., alcohols, poly alcohols, water), creams, lotions, ointments, oils, plasters, liposomes, powders, emulsions, microemulsions, and buffered solutions (e.g., hypotonic or buffered saline) or any other carrier known in the art for topically administering pharmaceuticals. A more complete listing of art-known carriers is provided by reference texts that are standard in the art, for example, Remington's Pharmaceutical Sciences, 16th Edition, 1980 and 17th Edition, 1985, both published by Mack Publishing Company, Easton, Pa., the disclosures of which are incorporated herein by reference in their entireties. In certain other embodiments, the topical formulations of the invention may comprise excipients. Any pharmaceutically acceptable excipient known in the art may be used to prepare the inventive pharmaceutically acceptable topical formulations. Examples of excipients that can be included in the topical formulations of the invention include, but are not limited to, preservatives, antioxidants, moisturizers, emollients, buffering agents, solubilizing agents, other penetration agents, skin protectants, surfactants, and propellants, and/or additional therapeutic agents used in combination to the inventive compound. Suitable preservatives include, but are not limited to, alcohols, quaternary amines, organic acids, parabens, and phenols. Suitable antioxidants include, but are not limited to, ascorbic acid and its esters, sodium bisulfite, butylated hydroxytoluene, butylated hydroxyanisole, tocopherols, and chelating agents like EDTA and citric acid. Suitable moisturizers include, but are not limited to, glycerine, sorbitol, polyethylene glycols, urea, and propylene glycol. Suitable buffering agents for use with the invention include, but are not limited to, citric, hydrochloric, and lactic acid buffers. Suitable solubilizing agents include, but are not limited to, quaternary ammonium chlorides, cyclodextrins, benzyl benzoate, lecithin, and polysorbates. Suitable skin protectants that can be used in the topical formulations of the invention include, but are not limited to, vitamin E oil, allatoin, dimethicone, glycerin, petrolatum, and zinc oxide.

It will also be appreciated that the pharmaceutical compositions of the present invention can be employed in combination therapies, that is, a compound and pharmaceutical compositions embodied herein, can be administered concurrently with, prior to, or subsequent to, one or more other desired therapeutics or medical procedures. The particular combination of therapies (therapeutics or procedures) to employ in a combination regimen will take into account compatibility of the desired therapeutics and/or procedures and the desired therapeutic effect to be achieved. It will also be appreciated that the therapies employed may achieve a desired effect for the same disorder, or they may achieve different effects (e.g., control of any adverse effects).

In certain embodiments, the pharmaceutically acceptable topical formulations of the invention comprise at least a compound of the invention and a penetration enhancing agent. The choice of topical formulation will depend or several factors, including the condition to be treated, the physicochemical characteristics of the inventive compound and other excipients present, their stability in the formulation, available manufacturing equipment, and costs constraints. As used herein the term "penetration enhancing agent" means an agent capable of transporting a pharmacologically active compound through the stratum corneum and into the epidermis or dermis, preferably, with little or no systemic absorption. A wide variety of compounds have been evaluated as to their effectiveness in enhancing the rate of penetration of drugs through the skin. See, for example, Percutaneous Penetration Enhancers, Maibach H. I. and Smith H. E. (eds.), CRC Press, Inc., Boca Raton, Fla. (1995), which surveys the use and testing of various skin penetration enhancers, and Buyuktimkin et ah, Chemical Means of Transdermal Drug Permeation Enhancement in Transdermal and Topical Drug Delivery Systems, Gosh T. K., Pfister W. R., Yum S. I. (Eds.), Interpharm Press Inc., Buffalo Grove, IU. (1997). In certain exemplary embodiments, penetration agents for use with the invention include, but are not limited to, triglycerides (e.g., soybean oil), aloe compositions (e.g., aloe-vera gel), ethyl alcohol, isopropyl alcohol, octolyphenylpolyethylene glycol, oleic acid, polyethylene glycol 400, propylene glycol, N-decylmethylsulfoxide. fatty acid esters (e.g., isopropyl myristate, methyl laurate, glycerol monooleate, and propylene glycol monooleate) and N-methylpyrrolidine.

In certain embodiments, the compositions may be in the form of ointments, pastes, creams, lotions, gels, powders, solutions, sprays, inhalants or patches. In certain exemplary embodiments, formulations of the compositions according to the invention are creams, which may further contain saturated or unsaturated fatty acids such as stearic acid, palmitic acid, oleic acid, palmito-oleic acid, cetyl or oleyl alcohols, stearic acid being particularly preferred. Creams of the invention may also contain a non-ionic surfactant, for example, polyoxy-40-stearate. In certain embodiments, the active component is admixed under sterile conditions with a pharmaceutically acceptable carrier and any needed preservatives or buffers as may be required. Ophthalmic formulation, eardrops, and eye drops are also contemplated as being within the scope of this invention. Additionally, the present invention contemplates the use of transdermal patches, which have the added advantage of providing controlled delivery of a compound to the body. Such dosage forms are made by dissolving or dispensing the compound in the proper medium. As discussed above, penetration enhancing agents can also be used to increase the flux of the compound across the skin. The rate can be controlled by either providing a rate controlling membrane or by dispersing the compound in a polymer matrix or gel.

### Kits

Kits are provided here, which comprise components in a package for ready usage in the methods according to the invention. Typically, written instructions to practice the methods of the invention will also be provided. The kits may include one or more MMPs, etc., media, and the like. Suitable buffers for diluting or reconstituting the active ingredients may also be provided. Some of the components may be provided in dry form, and may require reconstitution.

In embodiments, kits comprise one or more a matrix metalloproteinase (MMP), an inactive MMPs or a proenzyme (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, active fragments, mutants, variants, pharmaceutical compositions thereof, a pharmaceutically acceptable salt or prodrug thereof, or any combinations thereof. In some embodiments, kits comprise one or more MMPs, proMMPs, fragments and/or activating agents. Examples of activating agents, include, enzymes e.g. proteases which cleave the proMMPs into their active forms. In other examples, if an MMP- is inactivated, for example, by a modification of the MMP- active site e.g. glycosylation, etc., an agent which removes the modification would be an activating agent.

In some embodiments, kits for the isolation of stem cells comprise one or more MMPs, proMMPs, fragments and/or activating agents. Examples of activating agents, include, enzymes e.g. proteases which cleave the proMMPs into their active forms. In other examples, if an MMP is inactivated, for example, by a modification of the MMP active site e.g. glycosylation, etc., an agent which removes the modification would be an activating agent.

In other embodiments, kits for the reduction of fat deposits, catalysis of adipocyte tissues comprise one or more MMPs, proMMPs, fragments and/or activating agents.

All publications and patent documents cited in this application are incorporated by reference in pertinent part for all purposes to the same extent as if each individual publication or patent document were so individually denoted. By their citation of various references in this document, Applicants do not admit any particular reference is "prior art" to their invention.

The above disclosure generally describes the present invention. A more complete understanding can be obtained by reference to the following specific examples, which are provided herein for purposes of illustration only and are not intended to limit the scope of the invention.

### EXAMPLES

### Example 1: MMP-Induced Isolation of Stem Cells from Adipose Tissue.

Isolation of adipose derived stromal stem cells (ADSCs) has primarily been achieved with Collagenase I or Liberase, which is composed of collagenases I and II and thermolysin (Priya, Sarcar et al., (2012) J. Tissue Eng. Regen. Med. Jul. 27:1-9). Collagenases type I and II, are purified from the extremely dangerous bacillus *Clostridium,* an agent of gas gangrene. In addition, crude preparations from *Clostridium histolyticum* contain not only several collagenases but also a sulhydryl protease, clostripain, a trypsin-like enzyme, and an aminopeptidase. During Liberase enzyme production, collagenase isoenzymes are purified by a process that removes a significant amount of the endotoxin present in the raw material. There is a wide range of endotoxin contamination of traditional collagenase preparations compared with the endotoxin level of Liberase. However, regardless of the source, all purified collagenases and neutral proteases from bacterial bullion are contaminated with endotoxin (Priya, Sarcar et al., (2012) J. Tissue Eng. Regen. Med. Jul. 27:1-9). Prior studies have investigated the relative amount of endotoxin in different collagenase preparations and the impact on isolated cell health (Linetsky, E., L. Inverardi, et al. (1998). Transplantation Proc. 30: 345-346; Jahr, H., G. Pfeiffer, et al. (1999) J. Mol. Med. (Berl.) 77: 118-120; Salamone, M., G. Seidita, et al. (2010) Transplantation Proc. 42: 2043-2048) and observed that the presence of endotoxin is harmful for ADSC viability. In addition, success in cell transplantation is directly proportional to quality of stem cells isolated, cultivated, and allografts prepared.

A solution to the endotoxin contamination problem is the production of recombinant enzymes for use in ADSC isolation. A significant problem is that collagenase I is the most unstable component of Liberase, as the Ia form is rapidly autocatalytically degraded to the Ib form. Degraded collagenases have an adverse effect on islet viability (Brandhorst, H., N. Raimsch-Guenther, et al. (2008) Transplantation Proc. 40: 370-371).

Described herein, is the isolation of mesenchymal stem cells (MSCs) from adipose tissue utilizing MMP-3, MMP-9 and MMP-12 as compared to Collagenase I and LIBERASE™ (Hoffman-La Roche, Ltd.). The isolated MSCs were propagated in tissue culture and characterized morphologically and immunophetypically. To assure the MSC differentiation potency, the adipocyte cell differentiation was induced.

### Materials and Methods

*Enzymes*: A mixture of MMPs were used to achieve efficient catabolism of adipose tissue. A broad range of MMPs, including MMP-1, MMP-2, MMP-3, MMP-8, MMP-9, MMP-11, MMP-12, MMP-19 and MMP-25 were included. MMP-1 and MMP-8 were chosen based on their ability to efficiently cleave types I-III collagen. MMP-2 and MMP-9 cleave types IV and V collagen. MMP-3 and MMP-19 have activities towards type IV collagen, fibronectin, and laminin, MMP-12 cleaves elastin efficiently, and MMP-11 cleaves type VI collagen. This laboratory has produced recombinant MMP-1, MMP-3, MMP-8, MMP-9, and MMP-12 for many years. Recombinant production of other MMPs of interest (MMP-11, MMP-19 and MMP-25) can be synthesized in the inventors' laboratory.

*Enzyme activation*: Buffer reagents and chymotrypsin were obtained from Sigma (St. Louis, MO). LIBERASE™ was obtained from Roche (San Francisco, CA). Collagenase Type I was obtained from Worthington Biochemical (Lakewood, NJ). MMP-3 and MMP-12 were obtained from R&D Biosciences (San Diego, CA), while active MMP-9 was obtained from Calbiochem (Billerica, MA). MMP-3 was activated at 20 ng/µl concentration with 5 ng chymotrypsin/5 ng trypsin mixture for thirty minutes at 37°C. The reaction was stopped by addition of 2 mM PMSF (Biosynth, Itasca, IL). MMP-12 was self-activated for 30 hours in TSB (50 mM Tris, 150 mM NaCl, 10 mM CaCl₂, 1 µM ZnCl₂, 0.01% Brij-35, pH 7.5). Enzyme activity was tested at 125 ng/µl of MMP-3 and 20 ng/µl of MMP-12 with 5 µM Knight substrate in TSB. The Knight single-stranded peptide (SSP) [Mca-Lys-Pro-Leu-Gly-Leu-Lys(Dnp)-Ala-Arg-NH₂ (SEQ ID NO: 1)] was synthesized by methods described previously (Nagase, H., C. G. Fields, et al. (1994). J. Biol. Chem. 269: 20952-20957; Neumann, U., H. Kubota, et al. (2004). Anal. Biochem. 328: 166-173).

*Mesenchymal stem cell isolation from adipose tissue*: Lipoaspirate was collected from patients by liposuction. Tissue was washed once (50/50) with PBS. 2-10 ml of tissue was then digested with enzyme (400 ng/ml of adipose tissue of each MMP-, 120 U/ml adipose tissue Collagenase I and 0.45 Wunsch units/ml adipose tissue LIBERASE™ as a control) in TSB (50 mM Tris, 150 mM NaCl, 10 mM CaCl₂, 1 µM ZnCl₂, 0.01% Brij-35, pH 7.5) at 37°C for 60 min with intermittent shaking. Reaction was stopped by adding an equal volume of DMEM-LG/10% MSC-qualified FBS and centrifugation at 3000 x g for 20 min. The top fraction was discarded and the remaining Stromal Vascular Fraction (SVF) containing MSCs was resuspended in 20 ml DMEM-LG/10% FBS, filtered through 100 µm nylon mesh, and centrifuged at 1,200 x g for 20 min. Residual RBCs were removed with incubation of SVF in RBC lysis buffer (8.7 g/L ammonium chloride) for 10 min at 37°C. Cells were washed with DMEM-LG/10% FBS by centrifugation at 1,200 x g for 5 min. Cells were resuspended in DMEM-LG, 10% FBS, and 1 x penicillin/streptomycin, and plated at a density of 5000 cells/cm² into a tissue culture dish. Culture medium was changed after 1 d of cell adhesion. The medium was changed every 3 days until 70-80% cell confluence was achieved, at which point cells were de-adhered with TRYPLE™ reagent (Life Technologies, Carlsbad, CA) and passaged into MESENPRO RS™ medium (Life Technologies, Carlsbad, CA) at 200 cells/ cm².

*Evaluation of Isolated ADSCs-Viability*: Cells were stained with Trypan Blue and evaluated with Cellometer T4 Auto automatic cell counter (Nexcelom Bioscience, Lawrence, MA).

*Evaluation of Isolated ADSCs-Flow cytometry*: Flow cytometry experiments were performed at the VGTI Flow Cytometry Core Facility (Port St. Lucie, FL). MSCs were stained with BD STEMFLOW™ human MSC analysis kit (BD Biosciences, Franklin Lakes, NJ) which includes hMSC positive markers (CD73, CD90, and CD105), hMSC negative markers (CD11b, CD19, CD34, CD45, and HLA-DR) and isotype control antibodies. MSCs isolated by different methods and grown to passage 5 were stained with both positive, negative and isotype antibody staining controls in the dark at 4°C for 30 minutes. For each sample, 50,000 events were acquired on a BD LSR II analyzer (BD Biosciences, Franklin Lakes, NJ), and data were analyzed by Flowjo software.

*Evaluation of Isolated ADSCs-Adipogenic differentiation*: Adipogenesis was induced in confluent cultures using STEMPRO® Adipogenesis Differentiation Kit (Life Technologies, Carlsbad, CA) for 10 days. After 10 days cells were stained with 200 µl of Oil Red O solution (Sigma, St. Louis, MO) for 10 minutes at room temperature to detect oil droplet formation.

### Results

*Enzyme activation*: Commercially available recombinant MMP-3 and MMP-12 enzymes were activated according to manufacturer's instructions, while MMP-9 was purchased in an active form. The enzyme activity was tested using fluorescent Knight substrate produced in-house. All enzymes displayed comparable activities towards the Knight substrate (Figure 1).

*Isolation of MSCs:* Adipose tissue aspirates were treated either with Collagenase I, LIBERASE™, MMP-3, MMP-9 or MMP-12 for 30 minutes at 37°C. The SVF resulted in variable total cell numbers and displayed viability between 63.9% for MMP-3-treated samples up to 82.5% for MMP-12-treated samples (Table 1). Isolated SVF was plated in 10 cm³ tissue culture dishes at the density of 10⁴ cells/cm², and nonadherent cells were removed after 24 hours. The cells were cultured for 5-7 days to achieve 80% confluent passage 0 culture.

**Table 1. Isolated SVC total cell number and percent viability.**

| Enzyme | SVF cell number/ml adipose tissue | % viability |
|---|---|---|
| Collagenase I | 1.36 x 10⁶ | 74.0 |
| LIBERASE™ | 2.44 x 10⁶ | 70.0 |
| MMP-3 | 4.16 x 10⁵ | 63.9 |
| MMP-9 | 3.19 x 10⁵ | 73.4 |
| MMP-12 | 3.0 x 10⁶ | 82.5 |

Despite robust viability of the initial MMP-9-isolated SVF sample, we were unable to promote MSC growth in this sample. Since the SVF is a heterogeneous mixture of cells containing not only ADSCs but also preadipocytes, fibroblasts, resident monocytes, lymphocytes, vascular smooth muscle cells and others, the initial count and viability must have included all of the above cells with no ADSCs surviving the isolation. All other processed samples resulted in robust MSC culture with morphologically similar cells (Figure 2). Furthermore, after 5 passages, the cells from all samples (except for aforementioned MMP-9-isolate) retained excellent viability and similar morphology. No differences were observed in growth parameters such as doubling time and cell spreading among the different samples through passage 10. Due to similar characteristic of cells isolated by Collagenase I and LIBERASE™, further experiments shown here compared MMP-3, MMP-12 and LIBERASE™ isolated samples.

*Immunophenotypic characterization of MSCs:* Although there is no surface marker that uniquely defines MSCs, a common surface marker profile (e.g., CD34⁻, CD45⁻ (HSC markers), CD31⁻ (endothelial cell marker), CD44⁺, CD90⁺, CD73⁺ and CD105⁺) has been frequently used to define MSCs. Flow cytometry analysis of phenotypic MSC markers in samples isolated by LIBERASE™, MMP-3 and MMP-12 was performed at passage 5 (Figure 3). All samples were positive for MSC markers CD73, CD90 and CD105 at 99.8% or higher. Analysis of MSCs isolated using other MMPs produced similar results (Figures 7-10). Furthermore, MSCs in these samples displayed very similar levels of marker expression (Figure 4). Cells isolated by MMP-3 and LIBERASE™ were 100% positive for marker CD44, while sample isolated by MMP-12 did not yield reliable results for this marker due to low cell numbers in the sample. All samples were tested for the expression of MSC negative markers (CD11b, CD19, CD34, CD45, and HLA-DR) and isotype control antibodies with negative results.

*Adipogenesis*: Adipocyte differentiation potential of samples isolated by MMP-3, MMP-12, LIBERASE™ (Figure 5), and Collagenase I was detected by staining of lipid drop formation with oil red O. Adipogenesis was comparable in all samples as indicated by microscopic evaluation. Osteogenesis and chondrogenesis differentiation of MSCs following MMP-12 isolation was also observed (Figure 11).

### Discussion

Abundant reservoir and reliable isolation methods for obtaining mesenchymal stem cells are critical for successful application of these cells in future clinical uses. Since almost all stem cell applications require some degree of *ex vivo* expansion and manipulation before their targeted use, abundant source of the MSCs is necessary. Adipose tissue is an ideal source of these MSCs (ADSCs) as it is a ubiquitous and easily accessible source of adult stem cells with minimal patient discomfort, as opposed to other MSC sources, such as bone marrow. Isolation of these ADSCs must be easily accessible and cost-effective, as well as free of toxic byproducts that may harm the harvested cells. Isolation of ADSCs by using recombinant MMPs can address these requirements.

In this example, the isolation of ADSCs with MMP-3 and MMP-12, has been successfully demonstrated, and the morphology, phenotype and adipogenesis potential of ADSCs isolated with commonly used Collagenase I and LIBERASE™ was compared,

The morphological characteristics of ADSCs are: fibroblast like shape in culture, multipotent differentiation, extensive proliferation capacity, and a common surface marker profile (e.g., CD34⁻, CD45⁻ (HSC markers), CD31⁻ (endothelial cell marker), CD44⁺, CD90⁺, CD 73⁺ and CD105⁺). The ADSCs isolated by MMP-3 and MMP-12 displayed essentially identical morphological and phenotypical characteristics to cells isolated by bacterially-derived Collagenase I and LIBERASE™. Samples isolated with MMP-3, MMP-12 and LIBERASE™ had comparable levels of CD73, CD90 and CD105 as determined by flow cytometry, and were negative for negative markers. Furthermore, it was demonstrated that the adipogenic potential of the ADSCs isolated by MMP-3 and MMP-12 is retained as compared to cells isolated with LIBERASE™ and Collagenase I.

Interestingly, MMP-9 treatment of the adipose tissue did not yield any ADSCs. This could be due to the fact that MMP-9 is a gelatinase and is unable to access ADSCs residing within an intact collagenous ECM network contained within the adipose tissue. Further experiments include testing cocktails of MMPs and other MMPs (MMP-1, MMP-8, (for types I and III collagen), MMP-19 (for type IV collagen, fibronectin, and laminin), and MMP-11 (for digestion of type VI collagen)).

### Conclusions

The application of recombinant MMPs to isolate ADSCs described here is very significant, because without the highest quality of connective tissue degrading enzymes it is virtually impossible to liberate viable ADSCs without toxic byproducts. This innovative approach is expected to yield the following outcomes. First, a breakthrough in the entire field of the MSC isolation and transplantation technology will be achieved. Second, collection of MSCs of highest quality with a long time of life expectancy will be possible. Third, by abolishing enzyme toxicity, the standard protocol for adipose tissue processing and MSCs isolation will be developed. Fourth, a highly purified new recombinant MMP cocktail will be introduced in tissue dissociation practice, replacing current collagenases of microbial origin and becoming a new standard. Fifth, this approach may be applied to isolation of other cells, such as islet cells, where the current standard still relies on collagenases of bacterial origin. In conclusion, the research described here will have a significant impact on cell isolation from multiple tissue and organ origins.

### Example 2: Isolation of a Variety of Cells

*Islet Isolation from Pancreas:* There are three main steps: *In situ* pancreas perfusion with MMP- cocktail (e.g. two or more MMPs), pancreas digestion, and islet purification.

Mice are first anesthetized with Pentobarbital or similar and then moved to a hood. Each mouse is laid down with the abdominal side facing up and its skin is sterilized with 70% ethanol. An incision is made around the upper abdomen to expose the liver and intestines. The mouse ampulla is clamped with surgical clamps on the duodenum wall to block the bile pathway to the duodenum. 3 ml of buffer is made by dissolving MMP cocktail in 5 ml 1 × HBSS and is aspirated into a 5 ml syringe mounted with a 30G1/2-G needle. The needle is then inserted into the common bile duct through the joint site of the hepatic duct and the cystic duct and reaches the middle of common bile duct under the microscope. The solution is slowly injected to distend the pancreas. The pancreas is removed and placed in a 50 ml tube containing 2 ml of the above buffer. The tube is shaken briefly and then placed in a water bath at 37.5 °C for 15 min. After incubation, the tube is shaken by hand to disrupt the pancreas until the suspension turns homogeneous. Once the tissue suspension dissolves to very fine particles, the digestion is terminated by putting the tube on ice and adding 25 ml of 1 mM CaCl₂ in 1 × HBSS. This is centrifuged at 290g for 30 s at 4 °C and the supernatant discarded. Then, the pellet is resuspended with 20 ml ice-cold of 1 mM CaCl₂ in 1 × HBSS buffer, centrifuged again at 290g for 30 s at 4 °C, and the supernatant discarded. The resulting pellet is resuspended with 15 ml of 1 mM CaCl₂ in 1 × HBSS and then poured onto a pre-wetted 70 µm cell strainer. The tube is washed with 20 ml of 1 mM CaCl₂ again poured onto the strainer. Another 25 ml in 1 mM CaCl₂ in 1 × HBSS is poured through the filter. The strainer is turned upside down over a new petri dish and the captured islets are rinsed into the dish with 15 ml of buffer made up of L-glutamine (20 mM), penicillin (100 U ml⁻¹), streptomycin (100 µg ml⁻¹), and FBS (10%) into RPMI 1640 medium. The isolated islets are hand-picked using a pipette with a wide-open tip, counted and placed in 5% CO₂ incubator at 37 °C.

*Tissue dissociation to isolate a variety of cell types, including cardiac myocytes, fibroblasts, and dendritic cells:* Methods for preparing cell suspensions are: (a) Mechanically by mincing, sieving, or scratching off; (b) Chemically in the absence of divalent cations; and (c) Enzymatically by digesting with MMPs, collagenase, DISPASE®, trypsin, papain, elastase, pronase, hyaluronidase, or with selected combinations of these enzymes.

## Claims

1. A method in vitro of isolating islet cells from a pancreas or pancreatic tissue, comprising:
- contacting the pancreas or pancreatic tissue with a composition comprising an effective amount of at least one matrix metalloproteinase (MMP) or inactive MMPs or proenzymes (proMMPs) thereof, wherein the MMPs, inactive MMPs or proMMPs thereof, comprise: MMP-1, MMP-2, MMP-3, MMP-7, MMP-8, MMP-9, MMP-10, MMP-11, MMP-12, MMP-13, MMP-14, MMP-15, MMP-16, MMP-17, MMP-18, MMP-19, MMP-20, MMP-21, MMP-23A, MMP-23B, MMP-24, MMP-25, MMP-26, MMP-27, MMP-28, pharmaceutical compositions thereof, a pharmaceutically acceptable salt or prodrug thereof, or any combinations thereof, wherein the composition catabolizes or dissociates the pancreas or pancreatic tissue, thereby isolating the islet cells,
- administering one or more agents which activate the inactive MMPs or fragments thereof.

2. The method in vitro of claim 1, wherein the MMPs or fragments thereof, are active or inactive or combinations thereof.

3. The method in vitro of claim 1, comprising:
- contacting the pancreas or pancreatic tissue with a composition comprising an effective amount of two or more matrix metalloproteinases (MMPs) or inactive MMPs or proenzymes (proMMPs), pharmaceutical compositions thereof, a pharmaceutically acceptable salt thereof, or any combinations thereof.
